# EUROPEAN PATENT SPECIFICATION

(11) **EP 4 171 725 B1**
(45) Date of publication and mention of the grant of the patent: **19.11.2025**
(21) Application number: 21745415.6
(22) Date of filing: 24.06.2021
(51) Int. Cl.: A61N 1/362, A61N 1/368, A61N 1/365

(54) **TREATING CRT NON-RESPONDERS USING CARDIAC CONTRACTILITY MODULATION THERAPY**
BEHANDLUNG VON NICHT-RESPONDERN VON CRT MITTELS THERAPIE ZUR MODULATION DER HERZKONTRAKTILITÄT
TRAITEMENT DE NON-RÉPONDEURS À UNE CRT À L'AIDE D'UNE THÉRAPIE DE MODULATION DE LA CONTRACTILITÉ CARDIAQUE

(30) Priority: 25.06.2020 US 202063043814 P
(43) Date of publication of application: 03.05.2023
(73) Proprietor: Impulse Dynamics NV, Willemstad (CW)
(72) Inventor: PRUTCHI, David, Voorhees, New Jersey 08043 (US); KEDIKOGLOU, Simeon Ioannis, Tallahassee, Florida 32312 (US)
(74) Representative: Cohausz & Florack
(86) International application number: PCT/IB2021/055614
(87) International publication number: WO 2021/260620

(56) References cited:
- CA-A1- 2 589 409
- US-A1- 2010 069 985
- US-B1- 7 953 481

## Description

### FIELD AND BACKGROUND OF THE INVENTION

The present invention, in some embodiments thereof, relates to cardiac contractility modulation therapy and, more particularly, but not exclusively, to cardiac contractility modulation for cardiac resynchronization therapy (CRT) candidates.

Heart failure guidelines indicate a class I recommendation for CRT, only in patients with a QRS duration ≥130 ms and left bundle branch block (LBBB) pattern and a class II recommendation in patients with a QRS duration ≥130 ms and non-LBBB pattern.

Despite the careful selection criteria, approximately 30-50% of implanted patients fail to benefit from CRT, highlighting the need for alternative device therapies in this population.

U.S. patent number 210/069985 discloses "A method of manufacturing a part having an opening for receiving an insert, including forming the opening having an internal surface wherein the internal surface has a polygonal cross-section including a plurality of sides. The polygonal cross-section comprises a polygon having an area larger than a cross-section of the insert, and the sides constrain the insert so as to locate the insert in the opening when the insert is inserted into the opening."

U.S. patent number 7953481 discloses "A cardiac contractility modulating (CCM) device (30) includes an anti-arrhythmic therapy unit (38) for detecting a cardiac arrhythmia in a heart (2) of a patient based on processing electrical signals related to cardiac activity sensed at the heart, and for delivering anti-arrhythmic therapy to the heart. The device includes a cardiac contractility modulating (CCM) unit (40) capable of delivering cardiac contractility modulating (CCM) signals to the heart for modulating the contractility of a portion of the heart. The device (30) includes a power source (165). The device may be an implantable device or a non-implantable device. A method for delivering anti-arrhythmic therapy to a heart. The method includes applying one or more non-excitatory cardiac contractility modulating electrical signals to said heart."

### SUMMARY OF THE INVENTION

The invention provides a system variants of the present disclosure according to claim 1. Embodiments of the invention are provided in the dependent claims. For better understanding of the invention the present disclosure provides also further examples, not falling under the scope of the claims. In particular, the disclosed methods do not form part of the invention.

According to an aspect of some there is provided a method of selecting a patient for cardiac contractility modulation therapy, comprising: selecting a patient meeting a criteria for cardiac resynchronization therapy (CRT); detecting a potential difficulty in effective delivery of the CRT to the patient; and determining that the patient can benefit from cardiac contractility modulation therapy in spite of the potential difficulty.

In some variants of the present disclosure detecting comprises detecting whether an invasive treatment before CRT is expected to be effective.

In some variants of the present disclosure, detecting comprises receiving an indication that the patient suffers from one or more comorbidities selected from a list comprising renal insufficiency, diabetes mellitus, chronic obstructive pulmonary disease, sleeping disorders like apnea, and anemia.

In some embodiments, detecting comprises receiving an indication that the patient suffers from cardiac arrhythmia.

In some variants of the present disclosure, the cardiac arrhythmia comprises a non-left bundle branch block (non-LBBB).

In some variants of the present disclosure, determining comprises receiving an indication that the patient suffers from heart failure in NYHA classes II-IV.

In some variants of the present disclosure, , determining comprises receiving an indication that the patient suffers from a left ventricular ejection fraction in a range between 25% - 55%.

In some variants of the present disclosure, determining comprises receiving an indication that the patient exhibits a normal QRS duration, which is larger than 130 ms.

In some variants of the present disclosure, determining comprises setting a pulse generator of a cardiac device with parameters of a testing electric field for cardiac contractility modulation to be delivered through at least one electrode lead to the heart, and determining that an effect of the testing electric field on the patient indicates that the patient can benefit from cardiac contractility modulation therapy through the at least one electrode lead.

In some variants of the present disclosure, the testing electric field is a part of a testing session that lasts for up to 1 hour.

In some variants of the present disclosure, the pulse generator is configured to be located outside the body of the patient.

In some variants of the present disclosure, the method comprises selecting a cardiac tissue location suitable for delivery of cardiac contractility modulation and CRT therapy through the at least one electrode lead.

In some variants of the present disclosure, the at least one electrode lead comprises an anchor configured to anchor at least a portion of the at least one electrode lead to a septum of the heart.

In some variants of the present disclosure, the cardiac tissue location comprises the left ventricle or the right ventricle.

In some variants of the present disclosure, an electrode of the electrode lead suitable for delivery of both CRT and cardiac contractility modulation therapy has a surface area of at least 4 mm², and is coated with a high-capacitance and low-polarization coating.

In some variants of the present disclosure, the method comprises identifying that an effect of CRT is not a planned effect.

In some variants of the present disclosure, identifying comprises identifying that an effect of the CRT is an insufficient effect on increasing cardiac output.

In some variants of the present disclosure, identifying comprises identifying that the CRT effect comprises at least one undesired side effect.

In some variants of the present disclosure, the at least one undesired side effect comprises pain sensation.

In some variants of the present disclosure, the at least one undesired side effect comprises arrhythmia.

In some variants of the present disclosure, identifying comprises identifying at least 1 month following initiation of the CRT that the CRT effect is not a planned effect.

In some variants of the present disclosure, the method comprises selecting a treatment plan in which a dual purpose device is configured to generate electric fields for both CRT and cardiac contractility modulation therapy.

In some variants of the present disclosure, the dual purpose device is configured to time the CCM electric field during a refractory period of the heart as a train of biphasic bipolar pulses.

In some variants of the present disclosure, the CCM electric field has an amplitude of at least 5V, wherein a duration of each biphasic pulse is in a range of 9 milliseconds- 15 milliseconds.

In some variants of the present disclosure, the method comprises treating the patient with cardiac contractility modulation therapy according to the result of the determining.

In some variants of the present disclosure, treating comprises treating the patient with cardiac contractility modulation therapy in combination with CRT.

In some variants of the present disclosure, treating the patient with cardiac contractility modulation therapy comprises delivering an electric field during a refractory period of the right ventricle of the heart through at least one electrode lead anchored to the heart septum and wherein the treating the patient with CRT comprises delivering an electric field through at least one electrode lead positioned within the left ventricle, right ventricle or within a blood vessel located a distance of up to 1cm from the heart.

In some variants of the present disclosure, treating comprises delivering the electric field for CRT and cardiac contractility modulation therapy through the same electrode lead.

In some embodiments, treating comprises delivering an electric field for CRT within the same ventricle in which cardiac contractility modulation is delivered.

In some embodiments, treating comprises delivering an extended pacing signal for both CRT and cardiac contractility modulation therapy effects during a non-refractory period of the right ventricle.

In some variants of the present disclosure, the method comprises determining if treating the patient with the cardiac contractility modulation and the CRT has a desired effect on cardiac output and/or heart contraction; and modifying the CRT therapy based on the results of the determining.

According to an aspect of some variants of the present disclosure, there is provided a method of selecting a patient for cardiac contractility modulation therapy comprising:
identifying that an effect of CRT delivered is not a beneficial effect on the patient; and
modifying a setting of a cardiac device controller to generate a cardiac contractility modulation electric field based on the results of the identifying.

In some variants of the present disclosure, identifying comprises identifying that the CRT resulted with one or more unwanted side effects.

In some variants of the present disclosure, the side effect comprises pain or unpleasant feeling during or following CRT delivery.

In some variants of the present disclosure, identifying comprises identifying that delivery of CRT to the patient does not result with a beneficial effect on cardiac output.

In some variants of the present disclosure, identifying comprises identifying that the CRT delivery to the patient does not result with a beneficial effect on at least one of ejection fraction, intracardiac pressure, intra cardiac pressure gradient over a selected time period, NYHA class score, Peak VO2 and 6 minute walk score.

In some variants of the present disclosure, identifying comprises identifying that an effect of the delivered CRT comprises an insufficient effect on increasing intracardiac pressure or an insufficient effect on increasing intracardiac effect over a selected time period.

In some variants of the present disclosure, identifying comprising identifying arrhythmia in the patient following the CRT delivery.

In some variants of the present disclosure, the method comprises selecting at least one previously implanted electrode lead out of two or more previously implanted electrode leads, for delivery of the cardiac contractility modulation electric field.

In some variants of the present disclosure, the method comprises selecting at least one previously implanted electrode used for delivery of the CRT, for delivery of the cardiac contractility modulation electric field.

In some variants of the present disclosure, the selected at least one previously implanted electrode suitable for delivery of both CRT and cardiac contractility modulation therapy has a surface area of at least 4 mm², and is coated with a high-capacitance and low-polarization coating.

In some variants of the present disclosure, selecting comprises selecting at least one electrode lead positioned in at least one of the right ventricle, the left ventricle and within a blood vessel at a distance of less than 1 cm from the heart.

In some variants of the present disclosure, the selected at least one previously implanted electrode lead for cardiac contractility modulation is anchored to a septum of the heart.

In some variants of the present disclosure, the method comprises testing if the selected at least one previously implanted electrode lead is suitable for delivery the cardiac contractility modulation.

In some variants of the present disclosure, the method comprises signaling an implanted dual purpose device to generate the CRT electric field or the CCM electric field.

In some variants of the present disclosure, the method comprises replacing an implanted CRT device with a cardiac contractility modulation device.

According to an aspect of some variants of the present disclosure, there is provided a method for testing at least one electrode lead for delivery of cardiac contractility modulation therapy, comprising: determining to deliver CRT to a patient; implanting in the patient two or more electrode leads in the heart at locations suitable for delivery of the CRT; and testing if at least one electrode lead out of the two or more electrode leads is suitable for delivery of cardiac contractility modulation therapy.

In some variants of the present disclosure, testing comprises delivering an electric field with parameter values suitable for cardiac contractility modulation therapy through the at least one selected electrode lead, and testing whether the delivered electric field induces an undesired side effect.

In some variants of the present disclosure, delivering comprises delivering the electric field during the testing by a pulse generator located outside the body.

In some variants of the present disclosure, the undesired side effect comprises pain sensation and/or discomfort to the patient.

In some variants of the present disclosure, testing is performed during the implanting.

In some variants of the present disclosure, implanting comprises anchoring the at least one electrode lead to a septum of the heart, and wherein the testing comprises testing the at least one anchored electrode lead.

According to the invention there is provided a system for delivery of cardiac resynchronization therapy (CRT) and cardiac contractility modulation therapy comprising: a memory; a pulse generator configured to generate an electric field with parameter values suitable for CRT and cardiac contractility modulation; at least one electrode lead positioned within or at a distance of up to 1cm from the heart, electrically connected to the pulse generator;
a measurement circuitry connectable to at least one sensor, wherein the measurement circuitry is configured to measure at least one physiological parameter related to cardiac output; a control circuitry electrically connected to the measurement circuitry and the pulse generator; wherein the control circuitry is configured to signal the pulse generator to generate an electric field with parameter values suitable for CRT or cardiac contractility modulation, using electric field parameter values stored in the memory.

In some embodiments, the control circuitry is configured to determine whether an effect of an electric field delivered for CRT is a desired effect based on signals received from the measurement circuitry and by determining a relation between the signals or indications thereof and one or more desired effect indications stored in the memory.

In some embodiments, the system comprises a communication circuitry configured to transmit a wireless signal to a remote device located outside the body and wherein the control circuitry signals the communication circuitry to transmit the signal with instructions to provide cardiac contractility modulation therapy and/or with information related to the CRT effect, if the CRT effect is not a desired effect.

In some embodiments, the control circuitry receives instructions from a remote device to deliver cardiac contractility modulation therapy, via signals received by the communication circuitry.

In some embodiments, the control circuitry is configured to signal the pulse generator to generate an electric field with parameter values suitable for delivery of cardiac contractility modulation therapy to the heart if the CRT effect is not a desired effect.

In some embodiments, the control circuitry is configured to signal the pulse generator to generate the electric field with parameter values suitable for CRT and/or cardiac contractility modulation, and to deliver the electric field through the at least one electrode lead.

In some embodiments, at least one electrode of the at least one electrode lead, is suitable for delivery of both CRT and cardiac contractility modulation therapy, has a surface area of at least 4 mm², and is coated with a high-capacitance and low-polarization coating.

In the following, first to fifty-ninth configurations are described which pertain to the present disclosure but do not define the scope of the invention. Features from one of these configurations may be combined with one or more features from other ones of these configurations. It is expressly noted that the scope of the invention is solely defined by the claims appended to the present description.

A first configuration relates to a method for delivery of cardiac contractility modulation therapy, comprising:
selecting a patient meeting a criteria for cardiac resynchronization therapy (CRT);
detecting a potential difficulty in effective delivery said CRT to said patient;
determining that said patient could benefit from cardiac contractility modulation therapy in spite said potential difficulty;
treating said patient with cardiac contractility modulation therapy according to the result of said determining.

A second configuration, as a variant of the first configuration, relates to a method, wherein said detecting comprises detecting a potential need for an invasive treatment before CRT is expected to be effective.

A third configuration, as a variant of the first or second configuration, relates to a method, wherein said detecting comprises diagnosing said patient with one or more comorbidities selected from a list comprising renal insufficiency, diabetes mellitus, chronic obstructive pulmonary disease, sleeping disorders like apnea, and anemia.

A fourth configuration, as a variant of one of the first to third configurations, relates to a method, wherein said detecting comprises diagnosing said patient with cardiac arrhythmia.

A fifth configuration, as a variant of the fourth configuration, relates to a method, wherein said cardiac arrhythmia comprises a non-left bundle branch block (non-LBBB).

A sixth configuration, as a variant of one of the first to fifth configurations, relates to a method, wherein said determining comprises diagnosing the patient with heart failure in NYHA classes II-IV.

A seventh configuration, as a variant of one of the first to sixth configurations, relates to a method, wherein said determining comprises diagnosing the patient with a left ventricular ejection fraction in a range between 25% - 55%.

An eighth configuration, as a variant of the sixth or seventh configuration, relates to a method, wherein said determining comprises diagnosing the patient with normal QRS duration, which is larger than 130 ms.

A ninth configuration, as a variant of one of the first to eighth configurations, relates to a method, wherein said determining comprises delivering a testing electric field with cardiac contractility modulation parameters through at least one electrode lead to the heart and determining that an effect of said testing electric field an said patient indicates that said patient can benefit from cardiac contractility modulation therapy through said at least one electrode lead.

A tenth configuration, as a variant of the ninth configuration, relates to a method, wherein said testing electric field is delivered as part of a testing session that lasts for up to 1 hour.

An eleventh configuration, as a variant of the ninth or tenth configuration, relates to a method, wherein said testing electric field is delivered by a pulse generator located outside the body of said patient.

A twelfth configuration, as a variant of one of the first to eleventh configurations, relates to a method, comprising: implanting at least one electrode lead at a location suitable for delivery of cardiac contractility modulation and CRT therapy following said determining.

A thirteenth configuration, as a variant of the twelfth configuration, relates to a method, wherein said implanting comprises anchoring said at least one electrode lead to a septum of the heart.

A fourteenth configuration, as a variant of the twelfth or thirteenth configuration, relates to a method, wherein said implanting comprises implanting said at least one electrode lead within the left ventricle or the right ventricle.

A fifteenth configuration, as a variant of one of the twelfth to fourteenth configurations, relates to a method, wherein an electrode of said electrode lead suitable for delivery of both CRT and cardiac contractility modulation therapy has a surface area of at least 4 mm², and is coated with a high-capacitance and low-polarization coating.

A sixteenth configuration, as a variant of one of the twelfth to fifteenth configurations, relates to a method, comprising: delivering CRT to said patient following said detecting through said at least one electrode lead; identifying that an effect of said delivered CRT is not a planned effect on said patient, and wherein said treating comprises treating said patient with said cardiac contractility modulation therapy using said at least one electrode lead following said identifying.

A seventeenth configuration, as a variant of the sixteenth configuration, relates to a method, wherein said identifying comprises identifying that an effect of said delivered CRT is an insufficient effect on increasing cardiac output.

An eighteenth configuration, as a variant of the sixteenth or seventeenth configuration, relates to a method, wherein said identifying comprises identifying that said delivered CRT effect comprises at least one undesired side effect.

A nineteenth configuration, as a variant of the eighteenth configuration, relates to a method, wherein said at least one undesired side effect comprises pain sensation.

A twentieth configuration, as a variant of the eighteenth or nineteenth configuration, relates to a method, wherein said at least one undesired side effect comprises arrhythmia.

A twenty-first configuration, as a variant of one of the sixteenth to twentieth configurations, relates to a method, wherein said identifying comprises identifying at least 1 month following initiation of said CRT delivering that said CRT effect is not a planned effect.

A twenty-second configuration, as a variant of one of the twelfth to twenty-first configurations, relates to a method, wherein said implanting comprises implanting a dual purpose device configured to deliver both CRT and cardiac contractility modulation therapy.

A twenty-third configuration, as a variant of one of the first to twenty-second configurations, relates to a method, wherein said treating with cardiac contractility modulation therapy comprises delivering an electric field to the heart during a refractory period of the heart as a train of biphasic bipolar pulses.

A twenty-fourth configuration, as a variant of the twenty-third configuration, relates to a method, wherein said delivered electric field has an amplitude of at least 5V, and is transmitted as one or more biphasic pulses, wherein a duration of each biphasic pulse is in a range of 9 milliseconds - 15 milliseconds.

A twenty-fifth configuration, as a variant of one of the first to twenty-fourth configurations, relates to a method, wherein said treating comprises treating said patient with cardiac contractility modulation therapy in combination with CRT.

A twenty-sixth configuration, as a variant of the twenty-fifth configuration, relates to a method, wherein said treating said patient with cardiac contractility modulation therapy comprises delivering an electric field during a refractory period of the right ventricle of the heart through at least one electrode lead anchored to the heart septum and wherein said treating said patient with CRT comprises delivering an electric field through at least one electrode lead positioned within the left ventricle, right ventricle or within a blood vessel located a distance of up to 1cm from the heart.

A twenty-seventh configuration, as a variant of the twenty-sixth configuration, relates to a method, wherein said treating comprises delivering said electric field for CRT and cardiac contractility modulation therapy through the same electrode lead.

A twenty-eighth configuration, as a variant of one of the twenty-fifth to twenty-seventh configurations, relates to a method, wherein said treating comprises delivering an electric field for CRT within the same ventricle in which cardiac contractility modulation is delivered.

A twenty-ninth configuration, as a variant of one of the twenty-fifth to twenty-eighth configurations, relates to a method, wherein said treating comprises delivering an extended pacing signal for both CRT and cardiac contractility modulation therapy effects during a non-refractory period of the right ventricle .

A thirtieth configuration, as a variant of one of the twenty-fifth to twenty-ninth configurations, relates to a method, comprising:
determining if treating said patient with said cardiac contractility modulation and said CRT has a desired effect on cardiac output and/or heart contraction;
modifying said CRT therapy based on the results of said determining.

A thirty-first configuration relates to a method for delivery of cardiac contractility modulation therapy comprises:
delivering cardiac resynchronization therapy (CRT) to a patient;
identifying that an effect of said delivered CRT is not a beneficial effect on said patient;
treating said patient with cardiac contractility modulation therapy based on the results of said identifying.

A thirty-second configuration, as a variant of the thirty-first configuration, relates to a method, wherein said identifying comprises identifying that said CRT delivering to said patient results with one or more unwanted side effects.

A thirty-third configuration, as a variant of the thirty-second configuration, relates to a method, wherein said side effect comprises pain or unpleasant feeling during or following CRT delivery.

A thirty-forth configuration, as a variant of one of the thirty-first to thirty-third configuration, relates to a method, wherein said identifying comprises identifying that delivery of CRT to said patient does not result with a beneficial effect on cardiac output.

A thirty-fifth configuration, as a variant of one of the thirty-first to thirty-forth configuration, relates to a method, wherein said identifying comprises identifying that said CRT delivery to said patient does not result with a beneficial effect on at least one of ejection fraction, intracardiac pressure, intra cardiac pressure gradient over a selected time period, NYHA class score, Peak VO2 and 6 minute walk score.

A thirty-sixth configuration, as a variant of one of the thirty-first to thirty-fifth configuration, relates to a method, wherein said identifying comprises identifying that an effect of said delivered CRT comprises an insufficient effect on increasing intracardiac pressure or an insufficient effect on increasing intracardiac effect over a selected time period.

A thirty-seventh configuration, as a variant of one of the thirty-first to thirty-sixth configuration, relates to a method wherein said identifying comprising identifying arrhythmia in said patient following said CRT delivery.

A thirty-eighth configuration, as a variant of one of the thirty-first to thirty-seventh configuration, relates to a method, comprising selecting at least one previously implanted electrode lead out of two or more previously implanted electrode leads, for delivery of said cardiac contractility modulation therapy during said treating.

A thirty-ninth configuration, as a variant of one of the thirty-first to thirty-seventh configuration, relates to a method, comprising selecting at least one previously implanted electrode used for delivery of said CRT, for said cardiac contractility modulation therapy.

A fortieth configuration, as a variant of the thirty-ninth configuration, relates to a method, wherein said selected at least one previously implanted electrode suitable for delivery of both CRT and cardiac contractility modulation therapy has a surface area of at least 4 mm², and is coated with a high-capacitance and low-polarization coating.

A forty-first configuration, as a variant of the thirty-eighth or thirty-ninth configuration, relates to a method, wherein said selecting comprises selecting at least one electrode lead positioned in at least one of the right ventricle, the left ventricle and within a blood vessel at a distance of less than 1 cm from the heart.

A forty-second configuration, as a variant of one of the thirty-eighth to forty-first configurations, relates to a method, wherein said selected at least one previously implanted electrode lead for cardiac contractility modulation therapy is anchored to a septum of the heart.

A forty-third configuration, as a variant of one of the thirty-eighth to forty-second configurations, relates to a method, comprising:
testing if said selected at least one previously implanted electrode lead is suitable for delivery said cardiac contractility modulation therapy prior to said treating.

A forty-fourth configuration, as a variant of the forty-third configuration, relates to a method, wherein said testing comprises delivering an electric field with parameter values suitable for cardiac contractility modulation therapy through said selected at least one previously implanted electrode lead, and testing whether the delivered electric field induced cardiac contractility modulation therapy and/or caused pain or discomfort to the patient.

A forty-fifth configuration, as a variant of one of the thirty-first to forty-fourth configurations, relates to a method, comprising: implanting a dual purpose device configured to deliver both CRT and cardiac contractility modulation prior to said delivering, and wherein said treating comprises signaling said device to deliver said CRT therapy.

A forty-sixth configuration, as a variant of one of the thirty-first to forty-fourth configurations, relates to a method, comprising: implanting a CRT device prior to said delivering, and replacing said implanted CRT device with a cardiac contractility modulation device prior to said treating.

A forty-seventh configuration relates to a method for testing at least one electrode lead for delivery of cardiac contractility modulation therapy, comprising:
determining to deliver CRT to a patient;
implanting in said patient two or more electrode leads in the heart at locations suitable for delivery of said CRT;
testing if at least one electrode lead out of the two or more electrode leads is suitable for delivery of cardiac contractility modulation therapy.

A forty-eighth configuration, as a variant of the forty-seventh configuration, relates to a method , wherein said testing comprises delivering an electric field with parameter values suitable for cardiac contractility modulation therapy through said at least one selected electrode lead, and testing whether the delivered electric field induces an undesired side effect.

A forty-ninth configuration, as a variant of the forty-eighth configuration, relates to a method, wherein said delivering comprises delivering said electric field during said testing by a pulse generator located outside the body.

A fiftieth configuration, as a variant of the forty-eighth or forty-ninth configuration, relates to a method, wherein said undesired side effect comprises pain sensation and/or discomfort to said patient.

A fifty-first configuration, as a variant of one of the forty-seventh to fiftieth configurations, relates to a method, wherein said testing is performed during said implanting.

A fifty-second configuration, as a variant of one of the forty-seventh to fifty-first configurations, relates to a method, wherein said implanting comprises anchoring said at least one electrode lead to a septum of the heart, and wherein said testing comprises testing said at least one anchored electrode lead.

A fifty-third configuration relates to a system for delivery of cardiac resynchronization therapy (CRT) and cardiac contractility modulation therapy comprising:
a memory;
a pulse generator configured to generate an electric field with parameter values suitable for CRT and cardiac contractility modulation;
at least one electrode lead positioned within or at a distance of up to 1 cm from the heart, electrically connected to said pulse generator;
a measurement circuitry connectable to at least one sensor, wherein said measurement circuitry is configured to measure at least one physiological parameter related to cardiac output;
a control circuitry electrically connected to said measurement circuitry and said pulse generator; wherein said control circuitry is configured to signal said pulse generator to generate an electric field with parameter values suitable for CRT or cardiac contractility modulation, using electric field parameter values stored in said memory.

A fifty-fourth configuration, as a variant of the fifty-third configuration, relates to a system, wherein said control circuitry is configured to determine whether an effect of an electric field delivered for CRT is a desired effect based on signals received from the measurement circuitry and by determining a relation between said signals or indications thereof and one or more desired effect indications stored in said memory.

A fifty-fifth configuration, as a variant of the fifty-fourth configuration, relates to a system, comprising a communication circuitry configured to transmit a wireless signal to a remote device located outside the body and wherein said control circuitry signals said communication circuitry to transmit said signal with instructions to provide cardiac contractility modulation therapy and/or with information related to said CRT effect, if said CRT effect is not a desired effect.

A fifty-sixth configuration, as a variant of the fifty-fifth configuration, relates to a system, wherein said control circuitry receives instructions from a remote device to deliver cardiac contractility modulation therapy, via signals received by said communication circuitry.

A fifty-seventh configuration, as a variant of one of the fifty-forth to fifty-sixth configurations, relates to a system, wherein said control circuitry is configured to signal said pulse generator to generate an electric field with parameter values suitable for delivery of cardiac contractility modulation therapy to said heart if said CRT effect is not a desired effect.

A fifty-eighth configuration, as a variant of one of the fifty-third to fifty-seventh configurations, relates to a system, wherein said control circuitry is configured to signal said pulse generator to generate said electric field with parameter values suitable for CRT and/or cardiac contractility modulation, and to deliver said electric field through said at least one electrode lead.

A fifty-ninth configuration, as a variant of one of the fifty-third to fifty-eighth configurations, relates to a system, wherein at least one electrode of said at least one electrode lead, is suitable for delivery of both CRT and cardiac contractility modulation therapy, has a surface area of at least 4 mm², and is coated with a high-capacitance and low-polarization coating.

Unless otherwise defined, all technical and/or scientific terms used herein have the same meaning as commonly understood by one of ordinary skill in the art to which the invention pertains. Although methods and materials similar or equivalent to those described herein can be foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some variants of the present disclosure may be described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as identify that a CRT effect is not a desired effect might be expected to use completely different methods, e.g., making use of expert knowledge and/or the pattern recognition capabilities of the human brain, which would be vastly more efficient than manually going through the steps of the methods described herein.

### BRIEF DESCRIPTION OF THE SEVERAL VIEWS OF THE DRAWING(S)

Some variants of the present disclosure are herein described, by way of example only, with reference to the accompanying drawings. With specific reference now to the drawings in detail, it is stressed that the particulars shown are by way of example and for purposes of illustrative discussion of variants of the present disclosure. In this regard, the description taken with the drawings makes apparent to those skilled in the art how variants of the present disclosure may be practiced.

In the drawings:
FIG. 1A is a graph showing a cardiac contractility modulation pulse, according to some exemplary embodiments of the invention;
FIG. 1B is a flow chart of a process for identifying undesired response to CRT and delivery of cardiac contractility modulation therapy;
FIG. 2 is a schematic illustration showing locations of electrode leads in the heart suitable for delivery of cardiac contractility modulation therapy;
FIG. 3A is a flow chart of a process for treating a patient selected for CRT with cardiac contractility modulation therapy;
FIG. 3B is a flow chart of a process of identifying CRT non-responders subjects prior to implantation and delivery of cardiac contractility modulation therapy to these subjects;
FIGs. 4A and 4B are flow charts of a process for delivery of cardiac contractility modulation therapy when a pacing device, for example a CRT device is already implanted in the body;
FIG. 5 is a block diagram of a system for delivery of cardiac contractility modulation, according to some exemplary embodiments of the invention; and
FIG. 6 is a flow chart of a process for identifying an undesired response to CRT therapy and delivery of cardiac contractility modulation therapy by actions of a device;

### DESCRIPTION OF SPECIFIC EMBODIMENTS OF THE INVENTION

The present disclosure relates to cardiac contractility modulation therapy and, more particularly, but not exclusively, to cardiac contractility modulation for cardiac resynchronization therapy (CRT) candidates.

An aspect of some variants of the present disclosure relates to selecting patients for cardiac contractility modulation therapy. In some embodiments, the patients are candidates for a CRT treatment. In some embodiments, patients are selected for the cardiac contractility modulation therapy based on a probability that they will not respond to the CRT treatment, or that their response to the CRT treatment will not result with a desired therapeutic effect. In some embodiments, the patients are selected for the cardiac contractility modulation treatment prior to implantation of a cardiac pulse generator in their body.

As used herein in some embodiments of the invention, a CRT comprises providing synchronization treatment having typical configuration of at least 3 leads, for example a Right Atrial lead placed in the right atria, a Right ventricle lead that is placed in the right ventricle, in proximity to the ventricle septum, and a Left ventricle lead that is inserted through the coronary sinus into one of the left ventricle cardiac veins. In some embodiments, one or more of the CRT leads comprise at least one anchor, configured to anchor at least a portion of the lead, for example a distal portion of the lead, to cardiac tissue. In some embodiments, the at least one anchor comprises a screw, configured to allow screwing of the at least portion to the cardiac tissue. In some embodiments, one or more of the CRT leads are used to sense the electrical signal of the heart muscle and to pace the heart. In some embodiments, a pacing pulse, for example an electric field configured to generate pacing of the heart, has an amplitude of 3V-7V, for example 3V-6V, 4V-5.5V, 4.5V-6V, 5V-7V, 4V, 5V or any intermediate, smaller or larger value or range of values. In some embodiments, the delivered electric field has a duration, for example a pulse width, in a range of 0.1 milliseconds (ms)-0.8ms, for example 0.1ms-0.5ms, 0.3ms-0.6ms, 0.4ms-0.6ms, 0.5ms-0.6ms, 0.6ms-0.8ms or any intermediate, shorter or longer range of values. In some embodiments, the pulse width has a duration of at least 0.3ms, for example 0.4ms, 0.5ms, 0.6ms or any intermediate, shorter or longer time duration

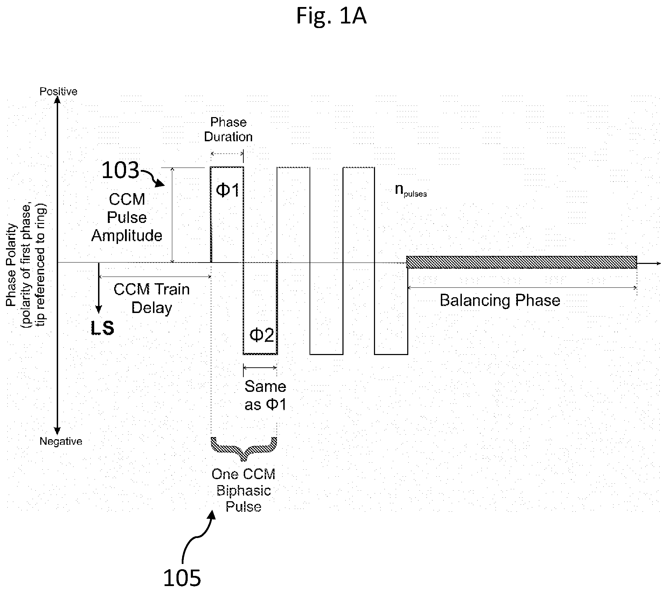

As used herein in some embodiments of the invention, a cardiac contractility modulation therapy comprises delivering of electric field pulses, for example as shown in fig. 1A, during a refractory period of the ventricles, for example a time period in which electrical signals cannot trigger new cardiac muscle contractions. In some embodiments, during cardiac contractility modulation therapy, one or more biphasic bipolar pulses is transmitted, for example as a train of biphasic pulses, from the same location, with an amplitude 103 of at least 4V, for example 5V, 6V, 7V, 8V, 9V or any intermediate, smaller or larger value. In some embodiments, a duration of each biphasic pulse 105 is in a range of 8-15 milliseconds (ms), for example 8-10 ms, 9-12 ms, 10-10.5ms, 10-11 ms, 10-12 ms, or any intermediate, smaller or larger range of values. In some embodiments, a train of 1, 2, 3, 4 or any intermediate, smaller or larger number of biphasic pulses is transmitted. In some embodiments, for example as shown in fig. 1A, a train of 3 consecutive biphasic pulses are delivered. In some embodiments, the cardiac contractility modulation therapy has parameters as described, for example in PCT application IB2020/050534.

According to some embodiments, CRT candidates are selected for cardiac contractility modulation based on at least one clinical parameter, for example history of atrial fibrillation (AF), diagnosed with ischemic cardiomyopathy, having lower percentage of CRT pacing, for example having an average ratio between CRT pacing beats and a number heart beats in a selected time period, is lower than 90%, for example lower than 80%, lower than 70% or any intermediate, smaller or larger value. In some embodiments, the selected time period for measuring the ration is at least 1 day, for example a day, a week, a month, 3 months or any intermediate, shorter or longer time period.

Alternatively or additionally, CRT candidates are selected for cardiac contractility modulation if they are diagnosed one, two or more comorbidities, for example renal insufficiency, diabetes mellitus, chronic obstructive pulmonary disease, sleeping disorders like apnea, and anemia. In some embodiments, CRT candidates are selected for cardiac contractility modulation based on results of a cardiac electrophysiological analysis, for example, subjects that exhibit non-left bundle branch block.

An aspect of some embodiments relates to treating patients receiving CRT with cardiac contractility modulation. In some embodiments, an activation mode of a device used to deliver CRT is changed to deliver cardiac contractility modulation. Alternatively, a device used to deliver CRT is replaced with a device configured to deliver cardiac contractility modulation. In some embodiments, patients receiving CRT are switched to receive cardiac contractility modulation therapy based on measurements of at least one clinical or physiological parameter related to heart contractility and/or propagation of electrical signals in the heart.

According to some embodiments, a device comprising a pulse generator, for example an implanted pulse generator (IPG), delivering CRT to a subject, measures at least one physiological parameter during and/or following delivery of an electric field pulse to the heart, indicating a response of the heart to the CRT. Alternatively or additionally, the at least one parameter is measured by a different device, for example a device implanted in the body or a device located outside the body. In some embodiments, the at least one physiological parameter is indicative of cardiac output and/or heart contraction. In some embodiments, the at least one physiological parameter comprises at least one of ejection fraction, cardiac output, intra cardiac pressure, intra cardiac pressure gradient over a selected time period, NYHA class score, Peak VO2, 6 min. walk score.

According to some embodiments, a controller of the device determines that a response of the subject to the delivered CRT, for example to the delivered electric field, is not a desired response, optionally based on the at least one measured physiological parameter. In some embodiments, if the determined response is not a desired response, the device modifies values of at least one activation parameter of the pulse generator, for example to allow delivery of cardiac contractility modulation therapy to the heart. In some embodiments, the at least one activation parameter comprises at least one of electric field intensity, delivery timing of the electric field with respect to heart contractility and/or electric field duration. Alternatively or additionally, the at least one activation parameter comprises number and/or location of electrodes to deliver the cardiac contractility modulation therapy.

According to some embodiments, if the determined response is not a desired response, then a device used to deliver CRT is replaced with a cardiac contractility modulation device configured to deliver cardiac contractility modulation therapy. In some embodiments, the cardiac contractility modulation device is connected to at least some of the electrode leads used to deliver CRT. Alternatively or additionally, at least one new electrode lead is connected to the cardiac contractility modulation and implanted, for example to position an electrode at the end of the electrode lead at a specific position in the heart, selected to provide cardiac contractility modulation. Optionally, two or more new electrode leads carrying electrodes are implanted in the body to provide the cardiac contractility modulation therapy. In some embodiments, existing implantable CRT ventricle leads are used for cardiac contractility modulation stimulation or cardiac contractility modulation testing.

According to some embodiments, CRT is delivered in combination with cardiac contractility modulation therapy, for example by a dual purpose device. In some embodiments, CRT is combined with cardiac contractility modulation therapy if a response, for example a physiological response or a clinical response of a subject to CRT alone is not a desired response. In some embodiments, cardiac contractility modulation therapy is delivered in synchronization with the CRT.

According to some embodiments, at least one of the electrode leads connected to the dual purpose device is used to deliver pacing, for example as part of the CRT, and at least one different electrode lead is used to deliver cardiac contractility modulation. Alternatively, at least one of the electrode leads connected to the dual purpose device is used to deliver both cardiac contractility modulation therapy and pacing at different time points.

According to some embodiments, cardiac contractility modulation is delivered by at least one electrode positioned in the right ventricle (RV) of the heart, and pacing is delivered by at least one different electrode positioned in the left ventricle (LV) of the heart. Alternatively, cardiac contractility modulation therapy is delivered by at least one electrode positioned in the RV, and at least one electrode positioned in the LV, and pacing is delivered by at least one different electrode in the LV, or by the same electrode positioned in the LV for delivering cardiac contractility modulation therapy. In some embodiments, both cardiac contractility modulation therapy and pacing are delivered to the RV and LV by different electrodes or by at least one shared electrode.

According to some embodiments, cardiac contractility modulation is delivered in synchronization with the delivery of pacing, and/or in synchronization with heart contraction. In some embodiments, and electric field for cardiac contractility modulation stimulation is generated with parameter values, for example intensity, amplitude and/or frequency suitable for a dual effect of pacing and cardiac contractility modulation in the same stimulation pulse. Optionally, the dual effect of pacing and cardiac contractility modulation is generated using at least one electrode lead, for example a CRT lead, positioned in a location in heart that is suitable for generating the dual effect on the heart.

According to some embodiments, cardiac contractility modulation is delivered after S wave timing shown in an electrocardiogram (ECG), that represents, for example, depolarization of Purkinje fibers. In some embodiments, cardiac contractility modulation is delivered up to 0.06 seconds, for example up to 0.04 seconds, up to 0.03 second, up to 0.02 seconds after detecting ventricle contraction. In some embodiments, at least one electrode lead positioned in the right ventricle is used to detect ventricle contraction and to deliver an electric field with parameter values suitable for cardiac contractility modulation therapy. In some embodiments, two or more electrod leads, for example 3 or 4 electrode leads in the right ventricle are used for sensing and delivery of cardiac contractility modulation pacing.

According to some embodiments, cardiac contractility modulation therapy is delivered during or following pacing in the LV, for example when combined with CRT. In some embodiments, cardiac contractility modulation is delivered up to 0.06 seconds, for example up to 0.04 seconds, up to 0.03 second, up to 0.02 seconds after detecting contraction of the right ventricle or following the delivery of CRT. In some embodiments, A potential advantage of delivering cardiac contractility modulation therapy during or following pacing in LV may be to promote cardiac contractility in the LV tissue.

According to some embodiments, CRT is added to a cardiac contractility modulation therapy delivered to the heart. In some embodiments, in case the addition of CRT does not improve heart contractility, then CRT is stopped while keeping cardiac contractility modulation therapy. In some embodiments, cardiac contractility modulation is delivered after CRT pacing at specific conditions, for example when a measured heart rate is higher than the patient average heart rate, for example by more than 0%, 5%, 10%, 20%, 40%, or any intermediate, smaller or larger value. Alternatively, or additionally, cardiac contractility modulation is delivered after CRT pacing during sleep or during specific sleep stages, for example deep sleep, Rapid Eye Movement (REM) sleep or non-REM sleep. Alternatively, or additionally, cardiac contractility modulation is delivered after CRT pacing if atrial arrhythmia is detected.

An aspect of some embodiments relates to using at least one existing previously implanted electrode lead, for example a CRT lead, for cardiac contractility modulation therapy. In some embodiments, at least one previously implanted electrode lead is selected from two or more previously implanted electrode leads for delivery of cardiac contractility modulation therapy. In some embodiments, the at least one electrode lead is selected by delivery of a testing electric field with parameters of cardiac contractility modulation therapy through the at least one electrode lead, and determining the effect of the delivered electric field. Alternatively, the testing electric field is delivered through two or more previously implanted electrode leads, and at least one electrode lead that is more suitable for delivery of cardiac contractility modulation therapy is selected out of the two or more previously implanted electreode leads. Optionally, at least one electrode lead previously used for CRT is selected for delivery cardiac contractility modulation therapy only or for delivery cardiac contractility modulation therapy in combination with CRT.

According to some embodiments, the appearance of undesired side effects, for example pain or unpleasant sensation following and/or during the electric field delivery is determined. In some embodiments, the at least one electrode lead for cardiac contractility modulation therapy is selected, optionally out of two or more electrode leads, based on the appearance of undesired side effects. In some embodiments, the at least one electrode lead is selected for cardiac contractility modulation is delivery of the testing electric field through the electrode did not cause undesired side effects, or caused tolerable undesired side effect in the patient. In some embodiments, if an undesired side effect is detected then at least one different electrode lead is selected for delivery of the testing electric field.

According to some embodiments, at least one electrode lead out of two or more electrode leads is selected to deliver cardiac contractility modulation based on measurements of at least one clinical or physiological parameter indicative of cardiac output and/or heart contraction following the delivery of the testing electric field.

According to some embodiments, at least one electrode lead, for example a CRT lead is implanted in advance in a location that can be used to deliver cardiac contractility modulation therapy. Optionally, two or more CRT leads, for example 2, 3, 4 CRT leads are implanted in advance in locations that can be used to deliver cardiac contractility modulation therapy. In some embodiments, the at least one location suitable to deliver cardiac contractility modulation comprises the right ventricle (RV), the left ventricle (LV), the right atrium (RA) or any combination of two or more CRT leads in these locations.

According to some exemplary embodiments, at least one electrode of an electrode lead configured to deliver both CRT and cardiac contractility modulation therapy is implanted in the RV. In some embodiments, the electrode has active fixation, for example using an electrically-active helix.

According to some embodiments, an electrode of an electrode lead configured to deliver both CRT and cardiac contractility modulation has a surface area of at least 2 mm², for example at least 4 mm², at least 6 mm² or any intermediate, smaller or larger surface area. Additionally or alternatively, the electrode is coated with a high-capacitance, low-polarization coating, for example titanium nitride or iridium oxide.

An aspect of some embodiments relates to delivering an electric field with parameter values suitable for cardiac contractility modulation therapy to induce pacing of the heart. In some embodiments, the electric field is delivered to patients that does not respond to CRT or to patients predicted not to respond to CRT. In some embodiments, an electric field with cardiac contractility modulation parameter values is delivered to induce pacing and promote cardiac contractility modulation therapy to the heart.

According to some embodiments, an electric field with cardiac contractility modulation parameter values is delivered to induce pacing by at least one electrode lead positioned in the right ventricle and/or in the left ventricle. In some embodiments, an extended pacing signal is delivered for said CRT and cardiac contractility modulation, for example as described in U.S Patent US7460907B1.

Before explaining at least one embodiment of the invention in detail, it is to be understood that the invention is not necessarily limited in its application to the details of construction and the arrangement of the components set forth in the following description and/or illustrated in the drawings and/or the Examples. The invention is capable of other embodiments or of being practiced or carried out in various ways.

### Exemplary general process for delivery of cardiac contractility modulation to patients not responding to CRT

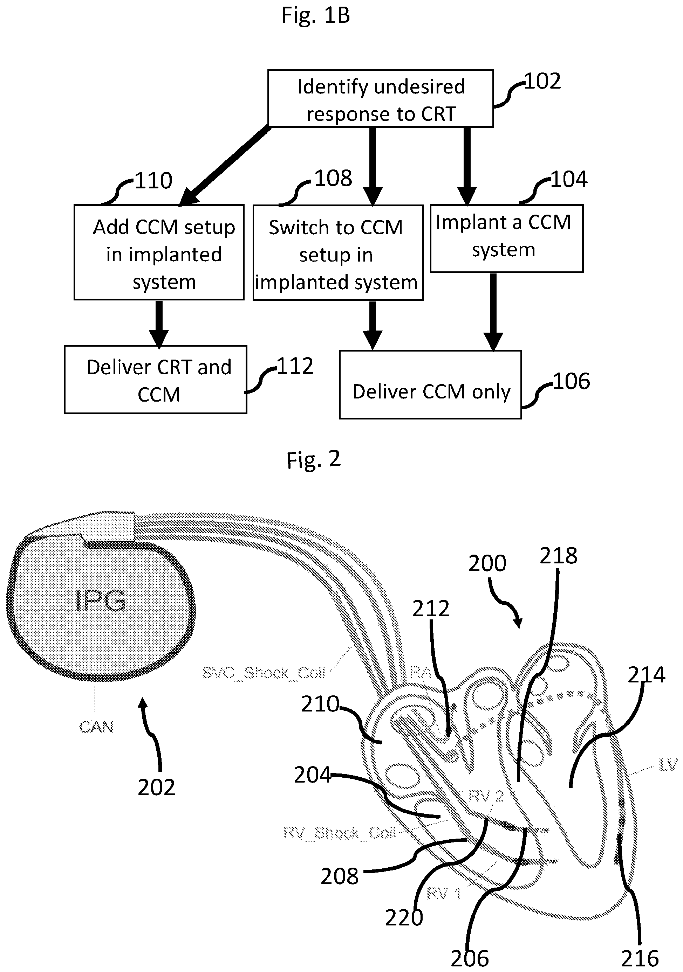

According to some exemplary embodiments, cardiac contractility modulation is delivered to patients that do not show a desired response to CRT treatment, for example patients that do not exhibit desired heart contractility following CRT. Reference is no made to fig. 1B, depicting a general process for delivery of cardiac contractility modulation therapy to patents that show undesired response to CRT, according to some exemplary embodiments of the invention.

According to some exemplary embodiments, an undesired response to CRT therapy is identified at block 102. In some embodiments, an undesired response is a response of the heart and/or body of the patient that indicates an effect, which is lower than a desired effect of the CRT therapy. In some embodiments, the undesired response is identified based on measurements of at least one physiological parameter related to heart activity.

According to some exemplary embodiments, an undesired effect of the CRT is identified by measuring at least one parameter related to cardiac output, for example ejection fraction of the heart, and/or stroke volume. In some embodiments, the undesired effect of CRT is detected at least 24 hours following CRT initiation, for example at least 1 week, at least 1 month or any intermediate, shorter or longer time period following CRT initiation.

According to some exemplary embodiments, a response to the CRT is measured from inside the body, for example by at least one implanted electrode. Optionally, the at least one implanted electrode is connected to an implanted CRT device. Alternatively, the at least one electrode is connected to a different measurement device, implanted inside the body or positioned outside the body.

According to some exemplary embodiments, the response to the CRT is measured from outside the body, for example by at least one electrode placed on the external surface of the skin. Alternatively, or additionally, the response to the CRT is measured by at least one imaging device, for example an ultrasound device, a magnetic resonance imaging (MRI) device, and/or a computerized tomography (CT) device. In some embodiments, the at least one imaging device, for example an ultrasound device, is implanted inside the body.

According to some variants of the present disclosure, when an undesired response to CRT is identified, a cardiac contractility modulation device configured to deliver cardiac contractility modulation therapy is implanted at block 104. In some variants of the present disclosure, the implanted cardiac contractility modulation device replaces an existing CRT device that is removed from the body of the patient. In some variants of the present disclosure, the newly implanted cardiac contractility modulation device is electrically connected to one or more CRT leads already implanted in the body, for example in the heart. Alternatively, the implanted cardiac contractility modulation device is implanted in addition to the existing CRT device already implanted in the body. In some variants of the present disclosure, the implanted cardiac contractility modulation device is electrically connected to at least one CRT lead, which is optionally also connected to the CRT device.

According to some variants of the present disclosure, the new implanted cardiac contractility modulation device delivers cardiac contractility modulation therapy at block 106.

According to some exemplary embodiments, when an undesired response to CRT is identified, a CRT device used to deliver the CRT is switched to a cardiac contractility modulation therapy activation protocol at block 108. In some embodiments, a cardiac contractility modulation therapy protocol is activated in a CRT device, for example in a dual-purpose device, at block 108. Alternatively, a different electrode lead connected to the CRT device is used to deliver cardiac contractility modulation therapy. Optionally, values of one or more parameters of an electric field are modified in order to provide the cardiac contractility modulation therapy, for example pulse duration, amplitude, and/or frequency.

According to some exemplary embodiments, when an undesired response to CRT is identified, at least one cardiac contractility modulation therapy protocol or parameters thereof is added to an already implanted CRT device, at block 110. In some embodiments, the at least one cardiac contractility modulation therapy protocol or parameters thereof are uploaded to an implanted CRT device from outside the body. In some embodiments, the implanted device is configured to deliver both CRT and cardiac contractility modulation in combination, for example pacing through at least one electrode lead, and cardiac contractility modulation through at least one different electrode lead, where both electrode leads are connected to the same device. In some embodiments, the CRT and cardiac contractility modulation therapy protocols are synchronized, for example a cardiac contractility modulation pulse is configured to be delivered shortly after pacing is delivered, and following left ventricle contraction. In some embodiments, a cardiac contractility modulation pulse is configured to be delivered shortly after pacing is delivered and during a refractory period of the heart.

According to some exemplary embodiments, CRT and cardiac contractility modulation therapy are delivered by the same device at block 112. In some embodiments, the CRT and the cardiac contractility modulation therapy are delivered in synchronization. Alternatively or additionally, the CRT and the cardiac contractility modulation therapy are delivered intermittently by the same device, for example by the same implantable device.

### Exemplary locations for cardiac contractility modulation therapy

According to some exemplary embodiments, one or more electrode leads are implanted in the heart. In some embodiments, one or more electrodes on the electrode leads are implanted in locations suitable for delivery of pacing, for example as part of CRT. In some embodiments, at least some of the locations of electrodes used for pacing are also suitable for delivery of cardiac contractility modulation. Reference is now made to fig. 2, depicting locations in the heart suitable for CRT and cardiac contractility modulation therapy, according to some exemplary embodiments of the invention.

According to some exemplary embodiments, at least one electrode lead, for example 2 electrode leads, 3 electrode leads, 3 electrode leads, 4 electrode leads, or any larger number of electrode leads are implanted in the heart. In some embodiments, each electrode lead comprises at least one electrode, for example 2, 3, 4, 5 or any larger number of electrodes. In some embodiments, at least some of the electrode leads, for example a distal end of the electrode leads is anchored to the heart tissue.

According to some exemplary embodiments, at least one electrode lead used for delivery of CRT is anchored within the heart 200, for example electrode lead 208. In some embodiments, at least one electrode lead used for delivery of CRT, for example lead 208 is anchored within a right ventricle of the heart 204. Alternatively or additionally, at least one electrode lead, for example lead 212 used for CRT is anchored within the right atrium 210. Alternatively or additionally at least one CRT electrode lead 216 is inserted through the coronary sinus, and is positioned to deliver an electric field to the left ventricle (LV).

According to some exemplary embodiments, CRT is delivered by at least one electrode lead positioned within the right ventricle 204, for example electrode lead 208 or 220, and optionally at least one electrode lead positioned within or near the left ventricle 214, for example electrode lead 216 inserted through the coronary sinus. Optionally, an electrode lead located within the heart ventricle 214 is used for cardiac contractility modulation, CRT or a combination of both CRT and cardiac contractility modulation therapy.

According to some exemplary embodiments, the CRT leads are electrically connected to an implanted pulse generator (IPG), for example IPG 202. In some embodiments, the IPG 202 is configured to deliver both cardiac contractility modulation and CRT through the electrode leads. In some embodiments, the IPG 202 is configured to deliver CRT and cardiac contractility modulation through the same lead. Alternatively, at least one lead connectd to the IPG 202 is used to deliver CRT, and at least one different electrode lead is used to deliver cardiac contractility modulation therapy. In some embodiments, at least one electrode lead, for example electrode lead 220 is used to deliver cardiac contractility modulation only.

According to some variants of the present disclosure, if an IPG is configured to deliver only CRT, then a cardiac contractility modulation IPG is implanted and is electrically connected to at least one electrode lead already implanted in the heart in a location suitable to deliver cardiac contractility modulation therapy. Alternatively, at least one new electrode lead for cardiac contractility modulation therapy is implanted, for example when implanting a cardiac contractility modulation IPG, in a preferred location in the heart or in surrounding blood vessels, for delivery of cardiac contractility modulation therapy.

According to some exemplary embodiments, at least one electrode lead is implanted in advance to allow delivery of cardiac contractility modulation therapy to the heart. In some embodiments, the at least one electrode lead suitable for delivery of cardiac contractility modulation therapy is implanted in advance within the right ventricle 204 or within the left ventricle 214. Alternatively or additionally, the at least one electrode lead suitable for delivery of cardiac contractility modulation therapy is implanted in advance within a blood vessel at a distance of up to 1cm, for example up to 0.5cm, up to 0.1 cm or any intermediate, smaller or larger distance from the heart. In some embodiments, the blood vessel comprises a vein of the heart, for example the coronary sinus. In some embodiments, the at least one electrode lead suitable for delivery of cardiac contractility modulation therapy is anchored in advance to the heart septum. In some embodiments, two or more electrode leads implanted in advance, for example when implanting a CRT device and/or CRT leads, are used to deliver cardiac contractility modulation therapy.

### Exemplary cardiac contractility modulation therapy for previously identified CRT non-responders

According to some variants of the present disclosure, patients that are candidates for CRT are evaluated, for example to determine a potential effect of the CRT in each patient. In some variants of the present disclosure, if the potential effect is lower than a desired effect, cardiac contractility modulation therapy is considered as a replacement to CRT or in combination with CRT. Optionally, if the potential effect of the CRT is a desired effect, then at least some of the implanted components, for example at least some of the implanted electrode leads can also be used to deliver cardiac contractility modulation therapy, in case a lower effect of the CRT is identified post implantation, for example during CRT delivery.

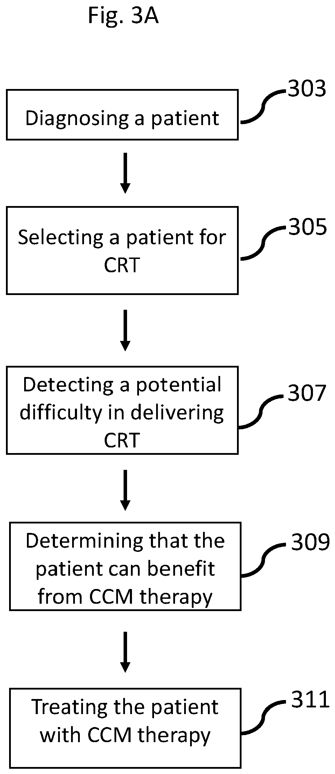

Reference is now made to fig. 3A depicting a process for treating a patient selected for CRT with cardiac contractility modulation therapy when a potential difficulty in delivering the CRT is detected, according to some variants of the present disclosure.

According to some variants of the present disclosure, a patient is diagnosed at block 303. In some variants of the present disclosure, the patient is diagnosed by measuring at least one physiological parameter related to the heart function, for example at least one parameter indicating cardiac output and/or cardiac contraction. Additionally or alternatively, an electrophysiological and/or an imaging analysis is performed for monitoring heart function, for example at least one of ECG, Echo imaging, CT and/or MRI. Additionally, during diagnosis, a medical history of the patient and/or the family of the patient is collected and examined.

According to some variants of the present disclosure, a patient is selected for CRT at block 305. In some embodiments, the patient is selected for CRT based on the results of the diagnosis performed at block 303. In some variants of the present disclosure, a patient is selected for CRT if the results of the diagnosis performed at block 303 indicate irregular heart contraction and/or insufficient cardiac output.

According to some variants of the present disclosure, a potential difficulty in the delivering the CRT treatment is detected at block 307. In some variants of the present disclosure, detecting a potential difficulty in delivering CRT comprises detecting that delivery of CRT will result with unwanted side effects, for example unpleasant sensation. Alternatively or additionally, detecting a potential difficulty comprises detecting that delivery of CRT to the patient will not result with a beneficial effect on heart failure condition parameters, for example will not result with a beneficial effect on heart beat and/or cardiac output. In some embodiments, detecting that delivery of CRT to the patient will not result with a beneficial effect comprises detecting that delivery of the CRT will results with an effect which is lower in at least 2%, for example at least 5%, at least 10%, at least 15%, at least 20% or any intermediate, smaller or larger percentage, from a desired beneficial effect.

According to some variants of the present disclosure, detecting a potential difficulty in delivering CRT comprises detecting that in order to benefit from the CRT, an invasive procedure needs to be performed on the patient, for example an ablative procedure. In some variants of the present disclosure, detecting a potential difficulty in delivering CRT comprises diagnosing the patient with at least one of atrial fibrillation (AF), ischemic cardiomyopathy, lower percentage of CRT pacing, for example having an average ratio between CRT pacing beats and a number heart beats in a selected time period, is lower than 90%, for example lower than 80%, lower than 70% or any intermediate, smaller or larger value. In some variants of the present disclosure, the selected time period for measuring the ratio is at least 1 day, for example a day, a week, a month, 3 months or any intermediate, shorter or longer time period.

According to some variants of the present disclosure, detecting a potential difficulty in delivering CRT comprises diagnosing the patient with one or more comorbidities selected from a list comprising renal insufficiency, diabetes mellitus, chronic obstructive pulmonary disease, sleeping disorders like apnea, and anemia. Alternatively or additionally, detecting a potential difficulty in delivering CRT comprises diagnosing the patient with cardiac arrhythmia, for example non-left bundle branch block (non-LBBB).

According to some variants of the present disclosure, detecting a potential difficulty in delivering CRT comprises detecting that delivery of CRT to the patient will not result with a beneficial effect on cardiac output, for example a desired increase in cardiac output of at least 0.5ml, for example at least 1ml, at least 3ml, at least 5ml or any intermediate, smaller or larger increase in cardiac output.

Alternatively or additionally, detecting a potential difficulty in delivering CRT comprises detecting that delivery of CRT to the patient will not result with a beneficial effect on ejection fraction, for example will not result with a desired increase of at least 0.5%, for example at least 1%, at least 3%, at least 5% or any intermediate, smaller or larger percentage of increase in ejection fraction.

Alternatively or additionally, detecting a potential difficulty in delivering CRT comprises detecting that delivery of CRT to the patient will not result with a beneficial effect on at least one of the following parameters, intracardiac pressure, intra cardiac pressure gradient over a selected time period, NYHA class score, Peak VO2 and/or 6 minute walk score. In some embodiments, a beneficial effect comprises an improvement in the parameters compared to a baseline value measured before CRT implantation and/or after CRT implantation.

According to some variants of the present disclosure, at block 309 it is determined if the patient can benefit from cardiac contractility modulation therapy. In some variants of the present disclosure, determining if the patient can benefit from cardiac contractility modulation therapy comprises diagnosing a patient with heart failure. In some variants of the present disclosure, determining that a patient can benefit from cardiac contractility modulation comprises diagnosing the patient with heart failure in NYHA classes II-IV. Alternatively or additionally, the patient is diagnosed with a left ventricular ejection fraction in a range between 25% - 55%, for example 25%-40%, 30%-45%, 40%-55% or any intermediate, smaller or larger range of values. In some embodiments, determining that a patient can benefit from cardiac contractility modulation comprises diagnosing the patient with normal QRS duration, for example QRS duration larger than 120 ms, for example larger than 130 ms, larger than 140 ms or any intermediate, smaller or larger value.

According to some variants of the present disclosure, the patient is treated with cardiac contractility modulation therapy at block 311. In some variants of the present disclosure, the patient is treated with cardiac contractility modulation therapy based on the results of the determining performed at block 311. In some variants of the present disclosure, a cardiac contractility modulation therapy comprises delivering of electric field pulses during a refractory period of the ventricles, for example a time period in which electrical signals cannot trigger new cardiac muscle contractions. In some variants of the present disclosure, during cardiac contractility modulation therapy, a train of biphasic bipolar pulses is transmitted from the same location, with an amplitude of at least 5V, for example 5V, 6V, 7V, 8V, 9V or any intermediate, smaller or larger value. In some variants of the present disclosure, the train of biphasic bipolar pulses is transmitted for a time period in a range of 8ms-13ms, for example 8ms-10ms, 9ms-10.5ms, 9.5ms-12ms, 9ms-13ms or any intermediate, smaller or larger range of values.

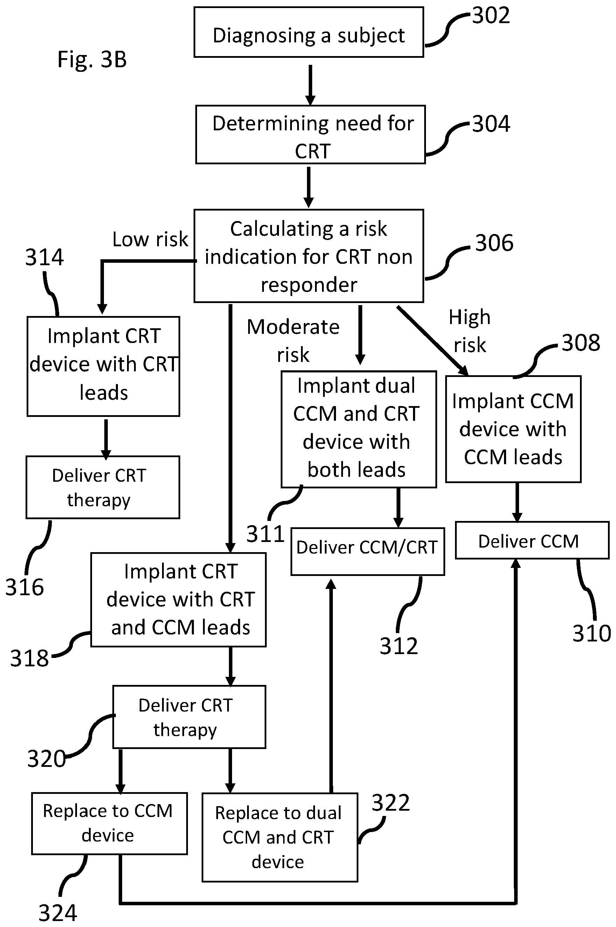

Reference is now made to fig. 3B, depicting a process for delivery of cardiac contractility modulation therapy to patients that were identified as CRT non-responders prior to CRT device implantation, according to some variants of the present disclosure.

According to some variants of the present disclosure, a subject is diagnosed at block 302. In some variants of the present disclosure, during subject diagnosis, clinical data and/or medical history is collected on the subject and optionally on the subject family. In some variants of the present disclosure, during diagnosis, at least one cardiac parameter is measured, for example electrical activity of the heart, contraction degree of at least part of the heart, for example contraction of the left ventricle, and/or heart rate. Alternatively, or additionally, during subject diagnosis, electrical conduction for example through heart tissue, and/or between the atria and ventricles, is measured. In some embodiments, during diagnosis information related to heart contraction pathologies, for example atrial fibrillation is collected. Alternatively or additionally, during diagnosis information related to heart failure is collected, for example QRS duration. In some variants of the present disclosure, at least some of the information is collected using at least one imaging technique, for example ultrasound, and/or echocardiography. Alternatively or additionally, at least some of the information is collected using electrocardiogram analysis.

According to some variants of the present disclosure, a subject is diagnosed with heart failure, at block 302. Additionally, the subject is diagnosed with a Left Bundle Branch Block (LBBB) with a QRS duration larger than or equal to 120 ms, for example 125 ms, 130 ms or any intermediate, smaller or larger value. Alternatively, the subject, for example the subject diagnosed with heart failure, is also diagnosed as non-LBBB with QRS duration larger than or equal to 150 ms, for example 155 ms, 160 ms, 170 ms or any intermediate, smaller or larger value. Additionally or alternatively, during diagnosis at block 302, the subject is classified according to the New York Heart Association (NYHA) stages of heart failure.

According to some variants of the present disclosure, a need of a subject to receive CRT is determined at block 304. In some variants of the present disclosure, a subject is selected for CRT based on the subject diagnosis performed at block 302. In some variants of the present disclosure, a subject with Left Bundle Branch Block (LBBB) with a QRS duration larger than 100 ms, for example larger than 110 ms, larger than 120 ms, larger than 130 ms or any intermediate, smaller or larger value of QRS duration is selected for CRT. In some embodiments, a subject in a New York Heart Association (NYHA) Classification II, III or IV, optionally non suffering from LBBB, with QRS duration larger than 130 ms, for example larger than 140 ms, larger than 150 ms, larger than 160 ms or any intermediate, smaller or larger value, is selected for CRT.

According to some variants of the present disclosure, a risk that a subject selected for CRT is a CRT non-responder, is estimated at block 306. In some variants of the present disclosure, a risk indicating that a subject selected for CRT is a CRT non-responder is calculated at block 306, for example using at least one of an algorithm and/or a lookup table, based on the data collected during subject diagnosis.

According to some variants of the present disclosure, a treatment plan is selected for the subject based on the calculated risk indication. In some variants of the present disclosure, if the calculated risk indication is low, for example lower than a predetermined value, a CRT device with CRT leads is implanted, for example to deliver CRT therapy. In some variants of the present disclosure, if the risk is very high, for example higher than a predetermined value, CRT is not considered as a potential treatment and a cardiac contractility modulation device with cardiac contractility modulation leads is implanted at 308, for example to deliver cardiac contractility modulation therapy only. In some variants of the present disclosure, if the risk indication is moderate, for example too high for delivery of only CRT and is lower than an indication for delivering of cardiac contractility modulation therapy only, then it is possible to implant a dual purpose device and to deliver both CRT and cardiac contractility modulation therapy at block 312. Alternatively, a CRT device with at least one cardiac contractility modulation lead is implanted at block 318, for delivery of CRT at block 320, while keeping the option to replace the device with a cardiac contractility modulation device or a dual purpose device, for delivery of cardiac contractility modulation therapy using the previously implanted electrode lead, in case CRT delivery is not effective.

According to some variants of the present disclosure, if a risk of a subject not to respond to CRT with a desired response is higher than a predetermined value, then a cardiac contractility modulation device is implanted in the subject at block 308. In some embodiments, the implanted cardiac contractility modulation device is configured to deliver only cardiac contractility modulation to the subject. In some variants of the present disclosure, at least one electrode lead, for example 2, 3, 4, 5 electrode leads configured to deliver cardiac contractility modulation therapy are implanted at locations that are favorable for delivery of cardiac contractility modulation therapy, for example an electrode lead anchored to the Right ventricle septum wall.

In some variants of the present disclosure, at least one of the implanted electrode leads is implanted in locations that are suitable for delivery of CRT.

According to some variants of the present disclosure, once the cardiac contractility modulation device and the electrode leads are implanted, cardiac contractility modulation therapy is delivered at block 310.

According to some variants of the present disclosure, if a risk of a subject not to respond to CRT with a desired response is higher than a predetermined value, then a dual purpose device configured to deliver both CRT and cardiac contractility modulation is implanted in the subject at block 311. In some variants of the present disclosure, the dual purpose device is implanted with at least one electrode lead configured to deliver CRT in a location suitable for delivery of CRT, and at least one additional electrode lead configured to deliver cardiac contractility modulation in a location suitable for delivery of cardiac contractility modulation therapy. Alternatively, the dual purpose device is implanted at block 311 with at least one electrode configured to deliver both CRT and cardiac contractility modulation therapy, in a location in the heart suitable for delivery both CRT and cardiac contractility modulation therapy for example in the right ventricle septum wall.

According to some variants of the present disclosure, once the dual purpose device and the at least one electrode lead is implanted, CRT and cardiac contractility modulation are delivered at block 312. Optionally, the CRT and cardiac contractility modulation therapy are delivered in synchronization. In some variants of the present disclosure, the CRT and cardiac contractility modulation therapy are delivered intermittently. Optionally, the CRT and cardiac contractility modulation therapy are delivered according to a contraction sequence of the heart, for example according to contraction timing of the atria and ventricles.

According to some variants of the present disclosure, if a risk of a subject not to respond to CRT with a desired response is high, for example higher than a predetermined value, then a CRT device with at least one electrode lead configured to deliver CRT and at least one electrode lead configured to deliver cardiac contractility modulation are implanted at block 318. In some variants of the present disclosure, the at least one electrode configured to deliver CRT is implanted in a location favorable for delivery of CRT to the patient heart. Additionally, the at least one electrode lead configured to deliver cardiac contractility modulation therapy is implanted in a location favorable for delivery of CC, therapy to the patient heart. Alternatively, at least one electrode lead, configured to deliver both CRT and cardiac contractility modulation therapy is implanted in a location favorable for both CRT and cardiac contractility modulation therapy, for example as described in fig. 2.

According to some variants of the present disclosure, CRT is delivered at block 320. In some variants of the present disclosure, CRT is delivered while monitoring the effect of the delivered CRT on heart contraction. In some variants of the present disclosure, an effect of the CRT on the heart of the patient is evident after at least 24 hours, at least 2 days, at least 1 week or any intermediate, shorter or longer time period, from initiating CRT delivery. In some variants of the present disclosure, evaluation of the delivered CRT effect is performed at least 1 month, for example at least 2, 3, 4, 5, 6, 7, 8 months or any intermediate, shorter or monger time period following the initial delivery of CRT. In some variants of the present disclosure, the effect of the delivered CRT is monitored by evaluating changes in heart failure parameters following CRT delivery, for example in comparison to a baseline value or any other reference.

According to some variants of the present disclosure, if the monitored CRT effect is lower than a desired effect, then the CRT device is replaced with a dual purpose device that is configured to deliver both CRT and cardiac contractility modulation therapy, at block 322. In some, the dual purpose device is electrically connected to one or more of the previously implanted electrode leads.

According to some variants of the present disclosure, if the monitored CRT effect is lower than a desired effect, then the CRT device is replaced with a device configured to deliver only cardiac contractility modulation, for example a cardiac contractility modulation device. In some variants of the present disclosure, the cardiac contractility modulation device is electrically connected to one or more of the previously implanted leads that are configured to deliver cardiac contractility modulation therapy, for example that are positioned in a location suitable for delivery of cardiac contractility modulation therapy.

According to some variants of the present disclosure, a decision whether to implant a cardiac contractility modulation only device or a dual purpose CRT and cardiac contractility modulation device, depends on the effect of CRT on the patient. In some variants of the present disclosure, if the CRT effect is not a desired effect but combining CRT and cardiac contractility modulation is expected to result with a desired effect, then a dual purpose device is implanted. In some variants of the present disclosure, if the CRT effect is lower than a desired effect and combining CRT with cardiac contractility modulation is not expected to result with a desired effect, then a cardiac contractility modulation only device is implanted.

According to some variants of the present disclosure, if the CRT effect is not a desired effect, and the clinical condition of the patient is expected to be unstable, then a dual purpose CRT and cardiac contractility modulation device is implanted, for example to allow better treatment flexibility in the future.

### Exemplary cardiac contractility modulation therapy following CRT delivery

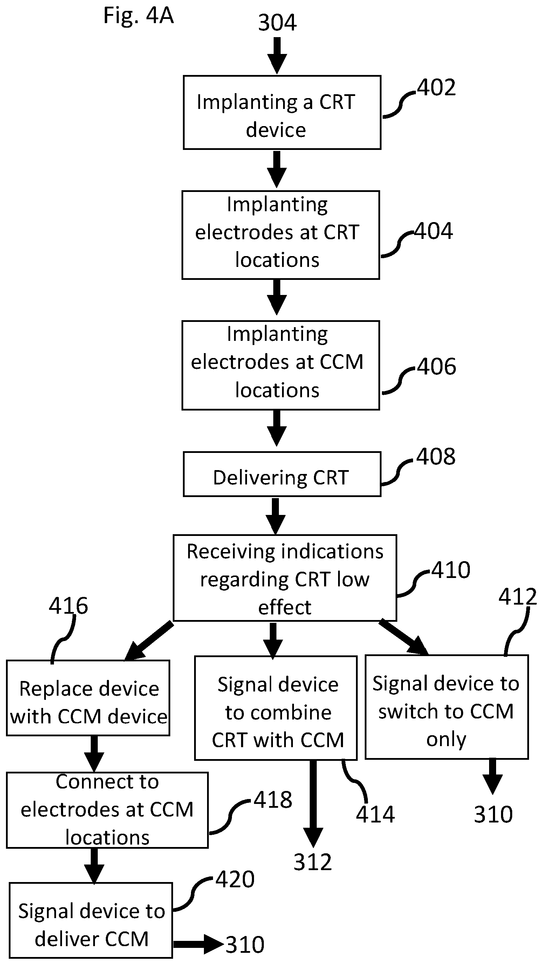

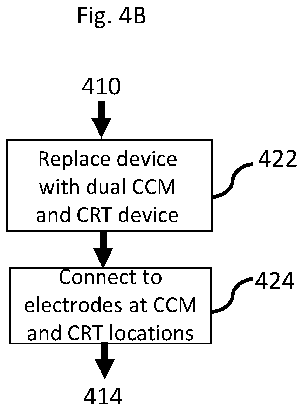

According to some variants of the present disclosure, a decision whether to provide cardiac contractility modulation therapy to a patient is made following delivery of CRT to the patient, and based on the results of the delivered CRT. Reference is now made to figs. 4A and 4B depicting a process for the delivery of cardiac contractility modulation to a patient already receiving CRT, according to some variants of the present disclosure.

According to some variants of the present disclosure, a subject is diagnosed and a need to receive CRT is determined at block 304.

According to some variants of the present disclosure, a CRT device is implanted at block 402. In some embodiments, the CRT device is configured to receive and transmit wireless signals to a device located outside the body.

According to some variants of the present disclosure, at least one electrode lead comprising one or more electrodes is implanted in locations suitable for delivery of CRT to the heart of the subject, at block 404.

According to some variants of the present disclosure, at least one electrode lead comprising one or more electrodes is implanted in locations suitable for delivery of cardiac contractility modulation therapy to the heart of the subject at block 406.

Alternatively or additionally, at least one electrode lead comprising one or more electrodes is implanted in locations suitable for delivery of CRT and cardiac contractility modulation to the heart.

According to some variants of the present disclosure, CRT is delivered to the heart at block 408. In some variants of the present disclosure, CRT is delivered through one or more of the electrodes implanted at block 404.

According to some variants of the present disclosure, indications regarding a low effect of the CRT are received at block 410. In some variants of the present disclosure, the indications are received based on measurements of at least one parameter related to the contraction of the heart, for example measurements performed from outside the body and/or from inside the body. In some embodiments, the indications are transmitted from the implanted CRT device to a device, for example a computer, and/or a cellular or a mobile phone located outside the body. In some embodiments, the indications are delivered to a remote server, and/or a remote cloud storage. In some embodiments, the indications are delivered to a physician, to a nurse or to a person monitoring a clinical condition of the patient.

According to some variants of the present disclosure, based on the received indications, if the implanted device is a dual purpose device configured to deliver CRT and cardiac contractility modulation therapy, signals are transmitted to the device to switch to cardiac contractility modulation therapy only. In some variants of the present disclosure, the signals delivered to the implanted device include at least one cardiac contractility modulation therapy protocol, values of at least one cardiac contractility modulation therapy parameter, for example electric field intensity, electric field frequency, duration of each pulse and/or information regarding synchronization of the cardiac contractility modulation therapy pulses with heart contraction and/or heart rhythm. In some variants of the present disclosure, the signals delivered to the implanted device at block 412 comprises information on which of the implanted electrodes to use when delivering the cardiac contractility modulation therapy. In some variants of the present disclosure, the signals delivered to the implanted device at block 412 include instructions to use at least one electrode implanted in a location suitable for cardiac contractility modulation therapy.

According to some variants of the present disclosure, based on the received indications, if the implanted device is a dual purpose device configured to deliver CRT and cardiac contractility modulation therapy, signals are transmitted to the device to combine cardiac contractility modulation therapy with CRT, at block 414. In some variants of the present disclosure, the signals delivered to the implanted device include at least one of a cardiac contractility modulation therapy protocol, values of at least one cardiac contractility modulation therapy parameter, for example electric field intensity, electric field frequency, duration of each pulse and/or information regarding synchronization of the cardiac contractility modulation therapy pulses with heart contraction, heart rhythm and/or with CRT. In some variants of the present disclosure, the signals delivered to the implanted device at block 414 include instructions regarding which of the electrodes or electrode leads to use for cardiac contractility modulation therapy and which of the electrodes or electrode leads to use for CRT.

According to some variants of the present disclosure, based on the received indications, the CRT device implanted at block 402 is replaced with a device that is configured to deliver cardiac contractility modulation only, at block 416. In some variants of the present disclosure, the CRT device is removed from the body. Alternatively, a cardiac contractility modulation device is implanted while keeping the CRT device in the body.

According to some variants of the present disclosure, the cardiac contractility modulation device is connected to at least one electrode lead, for example an electrode lead already implanted at block 406, positioned in a location suitable for delivery of cardiac contractility modulation.

According to some variants of the present disclosure, signals are delivered to the implanted cardiac contractility modulation to initiate cardiac contractility modulation therapy at block 420.

According to some variants of the present disclosure, based on the received indications, the CRT device implanted at block 402 is replaced with a dual purpose device that is configured to deliver CRT and cardiac contractility modulation therapy, at block 422. In some variants of the present disclosure, the dual purpose device is electrically connected to at least one electrode lead located at a position suitable for delivery of CRT, and at least one electrode lead located at a position suitable for delivery cardiac contractility modulation, at block 424. Optionally, the dual purpose device is electrically connected to at least one electrode lead located at a position that is suitable for delivery of both CRT and cardiac contractility modulation therapy.

### Exemplary device

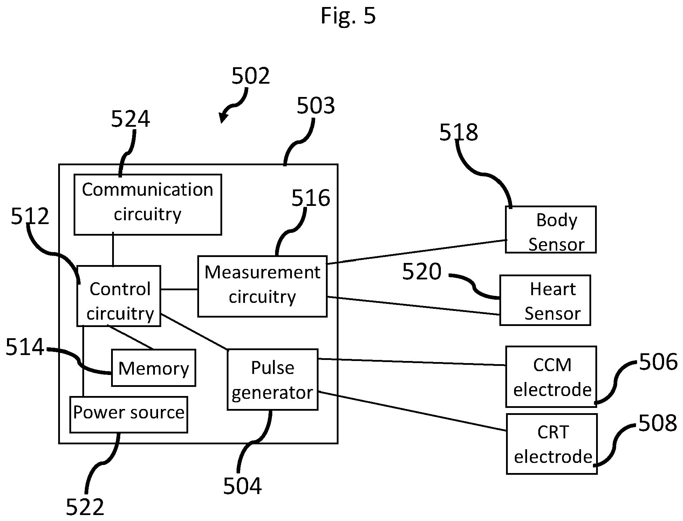

Reference is now made to fig. 5, depicting a device for delivery of cardiac contractility modulation therapy, according to some exemplary embodiments of the invention.

According to some exemplary embodiments, a device for delivery of cardiac contractility modulation therapy comprises an implantable pulse generator (IPG) unit, for example unit 502, electrically connected to at least one electrode lead positioned within the body. In some embodiments, the IPG unit 502 comprises at least one control circuitry 512 and at least one pulse generator 504, electrically connected to the control circuitry 512. In some embodiments, the pulse generator 504 is electrically connected to at least one, for example 2, 3, 4, 5, 6 or any larger number of electrode leads implanted in the body. In some embodiments, each electrode lead comprises at least one, for example 2, 3, 4, 5, 6 or any larger number of electrodes suitable for delivery of an electric field, for example as part of CRT and/or cardiac contractility modulation.

According to some exemplary embodiments, a casing of the IPG unit 502, for example casing 503 is shaped and sized to be implantable inside the body, for example subcutaneously implanted. In some embodiments, an external surface of the casing 503 is smooth, and is optionally planar. In some embodiments, the IPG unit is positioned outside the body, and is connected to at least one lead implanted inside the body.

According to some exemplary embodiments, the IPG unit 502 comprises a memory 514, for storing at least one cardiac contractility modulation protocol or parameters thereof. Additionally, the memory stores at least one CRT protocol or parameters thereof.

According to some exemplary embodiments, the pulse generator 504 is electrically connected to at least one electrode, for example cardiac contractility modulation electrode 506, configured to deliver cardiac contractility modulation therapy and positioned at a location suitable for delivery of cardiac contractility modulation therapy, for example as described in fig. 2. Additionally, the pulse generator 504 is electrically connected to at least one electrode, for example CRT electrode 508 configured to deliver CRT and positioned at a location suitable for delivery of CRT, for example as described in fig. 2.

According to some exemplary embodiments, the control circuitry 512 signals the pulse generator 504 to generate and deliver an electric field through the CRT electrode 508 with parameter values, for example electric field amplitude, electric field frequency, electric field pulse duration, suitable for CRT.

According to some exemplary embodiments, the control circuitry 512 signals the pulse generator 504 to generate and deliver an electric field through the cardiac contractility modulation electrode 506 with parameter values, for example electric field amplitude, electric field frequency, electric field pulse duration, suitable for cardiac contractility modulation therapy.

According to some exemplary embodiments, the IPG unit 502 comprises at least one measurement circuitry 516, electrically connected to the control circuitry 512. In some embodiments, the at least one measurement circuitry 516 is connected to at least one heart sensor, for example heart sensor 520, configured to record electrical signals, for example electrophysiological signals related to the heart activity. In some embodiments, the heart sensor 520 is configured to record signals related to heart contraction, for example contraction of at least one atrium, contraction of the left ventricle and/or contraction of the right ventricle. In some embodiments, the at least one heart sensor 520 is configured to record signals related to electrical conductance of heart tissue, for example heart tissue conductivity and/or heart tissue impedance.

According to some exemplary embodiments, the measurement circuitry 516 is connected to at least one additional sensor, for example body sensor 518, configured to record signals related to an electrophysiological parameter not related to heart activity, for example an implantable bio impedance sensor, intra cardiac pressure sensor that can measure cardiac performance parameters like ejection fraction, cardiac output, intra cardiac pressure, intra cardiac pressure gradient with time.

According to some exemplary embodiments, the control circuitry is configured to evaluate or determine an effect of CRT delivered by the IPG unit 502 to the heart, based on signals received from the sensors, 518 and 520 via the measurement circuitry 516. In some embodiments, the control circuitry 512 stores the signals or indications thereof, recorded from the electrodes in the memory 514.

According to some exemplary embodiments, the IPG unit 502 comprises a communication circuitry, for example communication circuitry 524, configured to receive and transmit signals, for example wireless signals from and to at least one external device located outside the body, for example a computer or a cellular device. In some embodiments, the communication circuitry 524 is configured to receive signals from at least one sensor located outside the body, for example at least one skin electrode. In some embodiments, the communication circuitry 524 is configured to receive signals from the external device, comprising at least one of a cardiac contractility modulation protocol, a CRT protocol, parameter values of an electric field used for CRT, parameter values of an electric field used for cardiac contractility modulation therapy. In some embodiments, information received via the communication circuitry 524 is stored in the memory 514.

According to some exemplary embodiments, the IPG unit 502 comprises a power source, for example power source 522. In some embodiments, the power source is a rechargeable power source, that can be recharged from outside the body, for example via wireless inductive charging. In some embodiments, the power source 522 is configured to deliver electric power to the IPG unit 502 components, for example to the pulse generator 504.

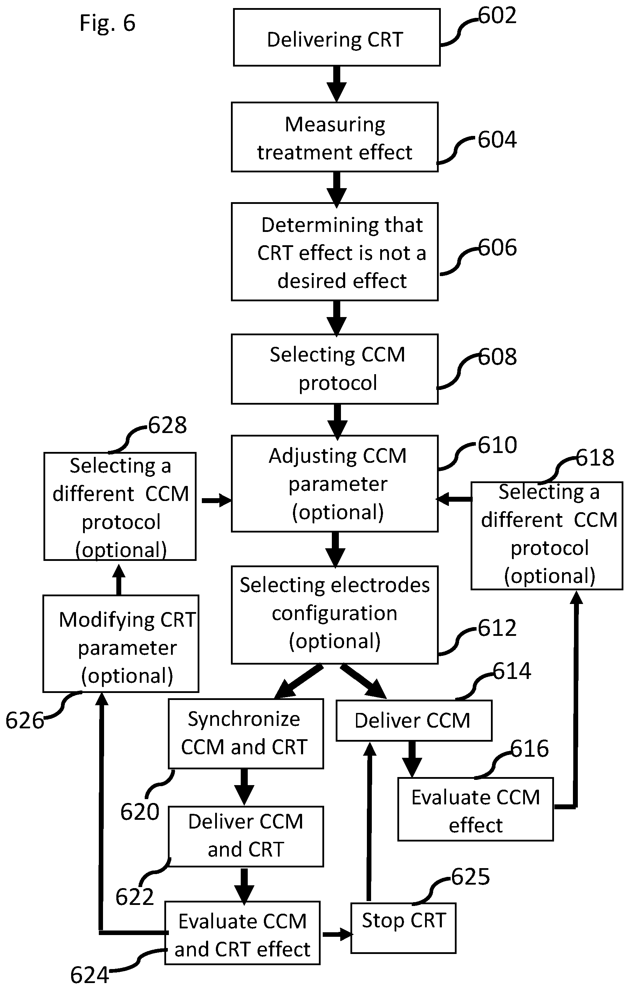

Reference is now made to fig. 6, describing activities of a device, for example activities performed by an IPG unit, according to some exemplary embodiments of the invention.

According to some exemplary embodiments, CRT is delivered at block 602. In some embodiments, the control circuitry 512 of the IPG unit 502 signals the pulse generator 504 to generate an electric field with parameter values selected for CRT. In some embodiments, the generated CRT electric field is delivered through at least one electrode, for example CRT electrode 508 to the heart.

According to some exemplary embodiments, an effect of the CRT, for example an effect of the CRT electric field is measured at block 604. In some embodiments, the control circuitry 512 measures the CRT effect based on signals received from the at least one heart sensor 520 and/or the at least body sensor 518. Optionally, the contro, circuitry 512 measured the CRT effect using at least one algorithm stored in the memory 514. In some embodiments, the CRT effect is measured by at least one external device located outside the body, for example an imaging device. In some embodiments, the results of the external device analysis are transmitted to the IPG unit 502 via the communication circuitry 524.

According to some exemplary embodiments, the control circuitry 512 determines that the measured CRT effect is not a desired effect, at block 606. In some embodiments, the control circuitry 512 determines that the measured CRT effect is not a desired effect by determining a relation between the measured CRT effect and at least one reference, for example a reference stored in the memory 514. In some embodiments, the control circuitry 512 determines that the measured CRT effect is not a desired effect using at least one algorithm or at least one lookup table stored in the memory 514.

According to some exemplary embodiments, the control circuitry 512 generates a score for the delivered CRT based on the measured CRT effect. In some embodiments, the control circuitry 512 determines that the CRT effect is not a desired effect based on the generated score, for example by determining a relation between the generated score and at least one reference value or a reference indication stored in the memory 514.

According to some exemplary embodiments, if the CRT effect is not a desired effect, then a cardiac contractility modulation protocol is selected at block 608. In some embodiments, the control circuitry 512 selects a cardiac contractility modulation protocol at block 608, for example a cardiac contractility modulation protocol stored in the memory 514. In some embodiments, the cardiac contractility modulation protocol is selected according to the measurements performed at block 604, according to the CRT treatment effect, and/or according to a clinical condition or physiolofical condition of the heart. In some embodiments, the cardiac contractility modulation protocol comprises at least one of parameter values of an electric field configured for cardiac contractility modulation therapy, at least one activation parameter of the pulse generator 504 and/or information regarding electrodes that can be used to deliver the cardiac contractility modulation electric field, for example electrodes position.

According to some exemplary embodiments, values of at least one parameter of the cardiac contractility modulation therapy are adjusted at block 610. In some embodiments, the at least one parameter comprises cardiac contractility modulation electric field parameter, and or a parameter of the cardiac contractility modulation protocol.

According to some exemplary embodiments, an electrodes configuration is selected, for example by the control circuitry 512, at block 612. In some embodiments, an electrodes configuration comprises a set of two or more electrodes positioned for delivery of the cardiac contractility modulation therapy out of a plurality of electrodes connected to the IPG unit, are selected at block 612. In some embodiments, at least one electrode out of two or more electrodes connected to the IPG unit, is selected for delivery of cardiac contractility modulation, for example based on the electrode location, electrode characteristics, and/or based on measurements of the CRT effect or other measurements of the clinical and physiological condition of the heart.

According to some exemplary embodiments, cardiac contractility modulation therapy is delivered at block 614. In some embodiments, cardiac contractility modulation therapy is delivered by signaling the pulse generator 504 to generate an electric field with parameter values selected for cardiac contractility modulation therapy, and/or to deliver the generated electric field through one or more of the electrodes selected at block 612. In some embodiments, the control circuitry 512 is configured to signal the pulse generator 504 to generate and deliver the cardiac contractility modulation electric field in synchronization with heart activity, for example in synchronization with contraction of the heart, and/or in synchronization with a depolarization state of at least one anatomical region of the heart. In some embodiments, the control circuitry 512 controls the timing of electric field generation and/or delivery based on signals received from one or more sensors measuring heart activity, for example heart sensor 520 shown in fig. 5.

According to some exemplary embodiments, an effect of the cardiac contractility modulation therapy on the heart is evaluated at block 616. In some embodiments, the effect of the cardiac contractility modulation therapy is evaluated based on signals received from at least one sensor, for example heart sensor 520. Alternatively, the effect of the cardiac contractility modulation therapy is evaluated based on measurements performed by at least one measurement device located outside the heart or outside the body. In some embodiments, the evaluation results or indications thereof are transmitted to the IPG unit 502, for example via the communication circuitry 524.

According to some exemplary embodiments, based on the results of the cardiac contractility modulation therapy evaluation performed at block 616, a different cardiac contractility modulation therapy protocol is optionally selected at block 618. Alternatively or additionally, based on the cardiac contractility modulation therapy evaluation results, values of at least one cardiac contractility modulation therapy parameter are optionally adjusted at block 610. Alternatively or additionally, based on the cardiac contractility modulation therapy evaluation results, at least one different electrode for delivery of the cardiac contractility modulation electric field is optionally selected or a different set of electrodes is optionally selected, at block 612.

According to some exemplary embodiments, instead of delivery of cardiac contractility modulation, a synchronization between CRT and cardiac contractility modulation therapy is performed at block 620. In some embodiments, the synchronization comprises timing synchronization between the delivery of an electric field with parameter values selected for CRT and delivery of an electric field with parameter values selected for cardiac contractility modulation therapy. Alternatively or additionally, the synchronization comprises synchronizing one or both CRT and cardiac contractility modulation therapy with heart activity, for example with the contraction of one or more of the heart compartments including left atrium, left ventricle, right atrium and right ventricle, and/or with the propagation of electric signals through the tissue of the heart. In some embodiments, the synchronization is performed by the control circuitry 512, optionally using at least one synchronization algorithm, at least one lookup table including one or more synchronization indications, stored in the memory 514.

According to some exemplary embodiments, CRT and cardiac contractility modulation therapy are delivered at block 622. In some embodiments, the control circuitry 512 signals the pulse generator 504 to generate a CRT electric field and a cardiac contractility modulation electric field, and/or to deliver them through at least one electrode to the heart tissue, according to the synchronization performed at block 620.

According to some exemplary embodiments, the effect of the dual treatment including CRT and cardiac contractility modulation therapy is evaluated at block 624. In some embodiments, the effect of the delivered CRT and cardiac contractility modulation therapy on the heart, for example on cardiac output and/or heart contractility, is evaluated at block 624. In some embodiments, the effect of the delivered CRT and cardiac contractility modulation therapy is evaluated based on signals received from at least one sensor connected to the IPG unit 502 and/or based on signals from at least one sensor or at least one device located outside the body, for example as described at blocks 604 and 616. In some embodiments, the control circuitry 512 performs the evaluation, for example automatically, based on information from at least one sensor connected to the IPG unit 502. Alternatively, the control circuitry 512 receives the evaluation results via the communication circuitry 524.

According to some exemplary embodiments, the effect of the delivered CRT and cardiac contractility modulation therapy is evaluated at block 624 at least 1 week, for example at least 1 month, at least 3 months, at least 6 months or any shorter or longer time period, following the treatment initiation. In some embodiments, the effect of the delivered CRT and cardiac contractility modulation comprises an effect on at least one of, ejection fraction, heart contractility, intracardiac pressure, intra cardiac pressure gradient over a selected time period, NYHA class score, Peak VO2 and 6 minute walk score.

According to some exemplary embodiments, if the evaluation results indicate that delivery of CRT does not result with a desired effect and/or that delivery of cardiac contractility modulation therapy is sufficient to reach a desired effect, CRT is stopped at block 625. In some embodiments, the control circuitry 512 signals the pulse generator to stop the generation of a CRT electric field, or stops signals to the pulse generator 504 to generate the CRT electric field, based on the evaluation results. Optionally, an indication signal is transmitted to an external device via the communication circuitry 524 if CRT is stopped.

According to some exemplary embodiments, values of at least one CRT parameter are optionally modified at block 626, for example by the control circuitry 512. In some embodiments, the values of the CRT parameter are modified based on the results of the evaluation performed at block 624. In some embodiments, synchronization between the delivered CRT and cardiac contractility modulation therapy is modified at block 626. Alternatively, a different CRT protocol is optionally selected.

According to some exemplary embodiments, a different cardiac contractility modulation protocol is optionally selected at block 628, for example by the control circuitry 512. In some embodiments, the different cardiac contractility modulation protocol is selected based on the results of the evaluation performed at block 624. Alternatively, values of at least one cardiac contractility modulation parameter are optionally adjusted at block 610, based on the results of the evaluation performed at block 628.

The terms "comprises", "comprising", "includes", "including", "has", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular forms "a", "an" and "the" include plural references unless the context clearly dictates otherwise.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations, which fall under the scope of the appended claims, are possible.

## Claims

1. A system for delivery of cardiac resynchronization therapy (CRT) and cardiac contractility modulation therapy to a human patient suffering from heart failure, comprising:
a memory (514);
a pulse generator (202, 504) configured to generate both an electric field with parameter values suitable for CRT and an electric field with parameter values suitable for cardiac contractility modulation; said parameter values including at least one of: electric field intensity, electric field frequency, delivery timing, and electric field duration;
at least one electrode lead (208, 216, 220, 506, 508) shaped and configured to be implanted within or at a distance of up to 1cm from the heart, electrically connected to said pulse generator;
a measurement circuitry (516) connectable to at least one sensor (518, 520), wherein said measurement circuitry (516) is configured to measure at least one physiological parameter related to cardiac output;
a control circuitry (512) electrically connected to said measurement circuitry (516) and said pulse generator;
**characterized in that**
based on said at least one physiological parameter measured by said measurement circuitry (516) said control circuitry (512) is configured to signal said pulse generator (202, 504) to generate an electric field with parameter values suitable for treating heart failure with CRT or with parameter values suitable for treating heart failure with cardiac contractility modulation, using electric field parameter values stored in said memory (514).

2. A system according to claim 1, wherein said control circuitry (512) is configured to determine whether an effect of an electric field delivered for CRT is a desired effect based on signals received from the measurement circuitry (516) and by determining a relation between said signals or indications thereof and one or more desired effect indications stored in said memory (514).

3. A system according to claim 2, comprising a communication circuitry (524) configured to transmit a wireless signal to a remote device located outside the body and wherein said control circuitry (512) signals said communication circuitry (524) to transmit said signal with instructions to provide cardiac contractility modulation therapy and/or with information related to said CRT effect, if said CRT effect determined based on signals received from the measurement circuitry
(516) is not a desired effect.

4. A system according to claim 3, wherein said control circuitry (512) receives instructions from a remote device to deliver cardiac contractility modulation therapy, via signals received by said communication circuitry (524).

5. A system according to any one of claims 2 to 4, wherein said control circuitry (512) is configured to signal said pulse generator (202, 504) to generate an electric field with parameter values suitable for delivery of cardiac contractility modulation therapy to said heart if said CRT effect determined based on signals received from the measurement circuitry (516) is not a desired effect.

6. A system according to any one of claims 1 to 5, wherein said control circuitry (512) is configured to signal said pulse generator (202, 504) to generate said electric field with parameter values suitable for CRT and/or cardiac contractility modulation, and to deliver said electric field through said at least one electrode lead (208, 220, 216, 506, 508).

7. A system according to any one of claims 1 to 6, wherein at least one electrode of said at least one electrode lead (208, 220, 216, 506, 508), is suitable for delivery of both CRT and cardiac contractility modulation therapy, has a surface area of at least 4 mm², and is coated with a high- capacitance and low-polarization coating.

8. A system according to claim 5, wherein said CRT effect determined based on signals received from the measurement circuitry (516) shows an insufficient effect on increasing cardiac output.

9. A system according to claim 5, wherein said CRT effect determined based on signals received from the measurement circuitry (516) includes an identified arrhythmia.

10. A system according to claim 1, wherein said at least one sensor (518, 520) includes an intra cardiac pressure sensor (518, 520) configured for measuring cardiac output.

11. A system according to claim 1, wherein said control circuitry (512) is configured to synchronize between delivery of an electric field with parameter values suitable for treating heart failure with CRT and an electric field with parameter values suitable for treating heart failure with cardiac contractility modulation.

## Patentansprüche

1. System zur Durchführung einer kardialen Resynchronisationstherapie (CRT) und einer kardialen Kontraktilitätsmodulationstherapie bei einem menschlichen Patienten mit Herzinsuffizienz, umfassend:
einen Speicher (514);
einen Impulsgenerator (202, 504), der so konfiguriert ist, dass er sowohl ein elektrisches Feld mit für die CRT geeigneten Parameterwerten und ein elektrisches Feld mit für die Modulation der Herzkontraktilität geeigneten Parameterwerten zu erzeugen; wobei die Parameterwerte mindestens einen der folgenden Werte umfassen: Intensität des elektrischen Felds, Frequenz des elektrischen Felds, Abgabe-Timing und Dauer des elektrischen Felds;
mindestens eine Elektrodenleitung (208, 216, 220, 506, 508), die so geformt und konfiguriert ist, dass sie im Herzen oder in einem Abstand von bis zu 1 cm vom Herzen implantiert werden kann und mit dem Impulsgenerator elektrisch verbunden ist;
eine Messschaltung (516), die mit mindestens einem Sensor (518, 520) verbunden werden kann, wobei die Messschaltung (516) so konfiguriert ist, dass sie mindestens einen physiologischen Parameter im Zusammenhang mit der Herzleistung zu messen;
eine Steuerschaltung (512), die mit der Messschaltung (516) und dem Impulsgenerator elektrisch verbunden ist;
**dadurch gekennzeichnet, dass**
die Steuerschaltung (512) dazu konfiguriert ist, auf der Grundlage des mindestens einen von der Messschaltung (516) gemessenen physiologischen Parameters dem Impulsgenerator (202, 504) zu signalisieren, ein elektrisches Feld mit Parameterwerten zu erzeugen, die für die Behandlung von Herzinsuffizienz mit CRT geeignet sind, oder mit Parameterwerten zu erzeugen, die für die Behandlung von Herzinsuffizienz mit kardialer Kontraktilitätsmodulation geeignet sind, wobei die im Speicher (514) gespeicherten elektrischen Feldparameterwerte verwendet werden.

2. System gemäß Anspruch 1, wobei die Steuerschaltung (512) so konfiguriert ist, dass sie auf der Grundlage von von der Messschaltung (516) empfangenen Signalen und durch Bestimmen einer Beziehung zwischen den Signalen oder Hinweisen hieraus und einer oder mehreren in dem Speicher (514) gespeicherten Hinweisen für gewünschte Effekte bestimmt, ob ein für CRT abgegebener Effekt ein gewünschter Effekt ist.

3. System gemäß Anspruch 2, das eine Kommunikationsschaltung (524) umfasst die so konfiguriert ist, dass sie ein drahtloses Signal an ein außerhalb des Körpers befindliches entferntes Gerät sendet, und wobei die Steuerschaltung (512) der Kommunikationsschaltung (524) signalisiert, das Signal mit Anweisungen zur Bereitstellung einer Therapie zur Modulation der Herzkontraktilität und/oder mit Informationen bezüglich des CRT-Effekts zu senden, wenn der auf der Grundlage von von der Messschaltung (516) empfangenen Signalen bestimmte CRT-Effekt kein gewünschter Effekt ist.

4. System nach Anspruch 3, wobei die Steuerschaltung (512) Anweisungen von einem entfernten Gerät zum Durchführen einer Herzkontraktilitätsmodulationstherapie über von der Kommunikationsschaltung (524) empfangene Signale empfängt.

5. System nach einem der Ansprüche 2 bis 4, wobei die Steuerschaltung (512) so konfiguriert ist, dass sie dem Impulsgenerator (202, 504) signalisiert, ein elektrisches Feld mit Parameterwerten zu erzeugen, die für die Durchführung einer Therapie zur Herzkontraktilitätsmodulation an dem Herzen geeignet sind, wenn der auf der Grundlage von von der Messschaltung (516) empfangenen Signalen bestimmte CRT-Effekt kein gewünschter Effekt ist.

6. System gemäß einem der Ansprüche 1 bis 5, wobei die Steuerschaltung (512) so konfiguriert ist, dass sie dem Impulsgenerator (202, 504) signalisiert, das elektrische Feld mit Parameterwerten zu erzeugen, die für CRT und/oder die Herzkontraktilitätsmodulation geeignet sind, und das elektrische Feld über die mindestens eine Elektrodenleitung (208, 220, 216, 506, 508) zu liefern.

7. System gemäß einem der Ansprüche 1 bis 6, wobei mindestens eine Elektrode der mindestens einen Elektrodenleitung (208, 220, 216, 506, 508) für die Abgabe sowohl einer CRT- als auch einer Herzkontraktilitätsmodulationstherapie geeignet ist, eine Oberfläche von mindestens 4 mm² aufweist und mit einer Beschichtung mit hoher Kapazität und geringer Polarisation beschichtet ist.

8. System gemäß Anspruch 5, wobei der auf der Grundlage der von der Messschaltung (516) empfangenen Signale bestimmte CRT-Effekt eine unzureichende Wirkung auf die Erhöhung der Herzleistung zeigt.

9. System gemäß Anspruch 5, wobei der auf der Grundlage von von der Messschaltung (516) empfangenen Signalen bestimmte CRT-Effekt eine identifizierte Arrhythmie umfasst.

10. System nach Anspruch 1, wobei der mindestens eine Sensor (518, 520) einen intrakardialen Drucksensor (518, 520) umfasst, der zum Messen der Herzleistung konfiguriert ist.

11. System gemäß Anspruch 1, wobei die Steuerschaltung (512) so konfiguriert ist, dass sie zwischen der Abgabe eines elektrischen Feldes mit Parameterwerten, die für die Behandlung von Herzinsuffizienz mit CRT geeignet sind, und einem elektrischen Feld mit Parameterwerten synchronisiert, die für die Behandlung von Herzinsuffizienz mit kardialer Kontraktilitätsmodulation geeignet sind.

## Revendications

1. Système pour administrer une thérapie de resynchronisation cardiaque (CRT) et une thérapie de modulation de contractilité (CRM) à un patient humain souffrant d'insuffisance cardiaque, comprenant :
une mémoire (514) ;
un générateur d'impulsions (202, 504) configuré pour générer à la fois un champ électrique avec des valeurs de paramètres adaptées à la CRT et un champ électrique avec des valeurs de paramètres adaptées à la modulation de la contractilité cardiaque ; lesdites valeurs de paramètres comprenant au moins l'une des valeurs suivantes : l'intensité du champ électrique, la fréquence du champ électrique, le timing d'administration et la durée du champ électrique ;
au moins un fil d'électrode (208, 216, 220, 506, 508) formé et configuré pour être implantée à l'intérieur du cœur ou à une distance maximale de 1 cm de celui-ci, connectée électriquement audit générateur d'impulsions ;
un circuit de mesure (516) pouvant être connecté à au moins un capteur (518, 520), dans lequel ledit circuit de mesure (516) est configuré pour mesurer au moins un paramètre physiologique lié au débit cardiaque ;
un circuit de commande (512) connecté électriquement audit circuit de mesure (516) et audit générateur d'impulsions ;
**caractérisé en ce que**
ledit circuit de commande (512) est configuré pour signaler, sur la base dudit au moins un paramètre physiologique mesuré par ledit circuit de mesure (516), audit générateur d'impulsions (202, 504) de générer un champ électrique avec des valeurs de paramètres adaptées au traitement de l'insuffisance cardiaque par CRT ou avec des valeurs de paramètres adaptées au traitement de l'insuffisance cardiaque par modulation de la contractilité cardiaque, en utilisant les valeurs de paramètres de champ électrique stockées dans ladite mémoire (514).

2. Système selon la revendication 1, dans lequel ledit circuit de commande (512) est configuré pour déterminer si un effet d'un champ électrique délivré pour la CRT est un effet souhaité sur la base de signaux reçus du circuit de mesure (516) et en déterminant une relation entre lesdits signaux ou indications de ceux-ci et une ou plusieurs indications d'effet souhaité stockées dans ladite mémoire (514).

3. Système selon la revendication 2, comprenant un circuit de communication (524) configuré pour transmettre un signal sans fil à un dispositif distant situé à l'extérieur du corps et dans lequel ledit circuit de commande (512) signale audit circuit de communication (524) de transmettre ledit signal avec des instructions pour fournir une thérapie de modulation de la contractilité cardiaque et/ou avec des informations relatives audit effet CRT, si ledit effet CRT déterminé sur la base des signaux reçus du circuit de mesure (516) n'est pas un effet souhaité.

4. Système selon la revendication 3, dans lequel ledit circuit de commande (512) reçoit des instructions d'un dispositif distant pour administrer une thérapie de modulation de la contractilité cardiaque, via des signaux reçus par ledit circuit de communication (524).

5. Système selon l'une quelconque des revendications 2 à 4, dans lequel ledit circuit de commande (512) est configuré pour signaler audit générateur d'impulsions (202, 504) de générer un champ électrique avec des valeurs de paramètres adaptées à l'administration d'un traitement de modulation de la contractilité cardiaque audit cœur si ledit effet CRT déterminé sur la base des signaux reçus du circuit de mesure (516) n'est pas un effet souhaité.

6. Système selon l'une quelconque des revendications 1 à 5, dans lequel ledit circuit de commande (512) est configuré pour signaler audit générateur d'impulsions (202, 504) de générer ledit champ électrique avec des valeurs de paramètres adaptées à la CRT et/ou à la modulation de la contractilité cardiaque, et pour délivrer ledit champ électrique par l'intermédiaire d'au moins une électrode (208, 220, 216, 506, 508).

7. Système selon l'une quelconque des revendications 1 à 6, dans lequel au moins une électrode dudit au moins un fil d'électrode (208, 220, 216, 506, 508) est adaptée à la délivrance d'une thérapie de CRT aussi que d'une thérapie de modulation de la contractilité cardiaque, a une surface d'au moins 4 mm² et est recouverte d'un revêtement à haute capacité et faible polarisation.

8. Système selon la revendication 5, dans lequel ledit effet CRT déterminé sur la base des signaux reçus du circuit de mesure (516) montre un effet insuffisant sur l'augmentation du débit cardiaque.

9. Système selon la revendication 5, dans lequel ledit effet CRT déterminé sur la base des signaux reçus du circuit de mesure (516) comprend une arythmie identifiée.

10. Système selon la revendication 1, dans lequel ledit au moins un capteur (518, 520) comprend un capteur de pression intracardiaque (518, 520) configuré pour mesurer le débit cardiaque.

11. Système selon la revendication 1, dans lequel ledit circuit de commande (512) est configuré pour synchroniser entre la délivrance d'un champ électrique avec des valeurs de paramètres adaptées au traitement de l'insuffisance cardiaque par CRT et un champ électrique avec des valeurs de paramètres adaptées au traitement de l'insuffisance cardiaque par modulation de la contractilité cardiaque.
